# EUROPEAN PATENT SPECIFICATION

(11) **EP 2 892 577 B1**
(45) Date of publication and mention of the grant of the patent: **30.01.2019**
(21) Application number: 13835980.7
(22) Date of filing: 03.09.2013
(51) Int. Cl.: A61L 27/38

(54) **METHODS OF TISSUE GENERATION**
VERFAHREN ZUR GEWEBEGENERATION
PROCÉDÉS DE GÉNÉRATION DE TISSU

(30) Priority: 04.09.2012 US 201261696487 P
(43) Date of publication of application: 15.07.2015
(73) Proprietor: Anthrogenesis Corporation, Warren, NJ 07059 (US)
(72) Inventor: BHATIA, Mohit B., Manalapan, NJ 07726 (US); HARIRI, Robert J., Bernardsville, NJ 07924 (US); HOFGARTNER, Wolfgang, Florham Park, NJ 07932 (US); WANG, Jia-Lun, Cherry Hill, NJ 08003 (US); YE, Qian, Livingston, NJ 07039 (US)
(74) Representative: Mewburn Ellis LLP
(86) International application number: PCT/US2013/057806
(87) International publication number: WO 2014/039429

(56) References cited:
- WO-A1-2009/111030
- WO-A2-2008/057162
- WO-A2-2013/096741
- US-A1- 2003 091 543
- US-A1- 2007 299 537
- US-A1- 2008 070 304
- US-A1- 2009 053 805
- US-A1- 2010 124 563
- US-A1- 2011 250 688
- A. Skardal et al.: "Bioprinted amniotic fluid derived stem cells accelerate wound healing in skin", JOURNAL OF TISSUE ENGINEERING AND REGENERATIVE MEDICINE, vol. 6 (Suppl. 1), 61.P37, 3 September 2012 (2012-09-03), 2012, page 376, XP002754769, DOI: 10.1002/term.1586 Retrieved from the Internet: URL:http://onlinelibrary.wiley.com/doi/10. 1002/term.1586/epdf [retrieved on 2016-02-25]
- R. Hariri: "Human placental extracellular matrix as a platform for novel tissue engineered products", JOURNAL OF TISSUE ENGINEERING AND REGENERATIVE MEDICINE, vol. 6 (Suppl. 1), 76.06, 3 September 2012 (2012-09-03), 2012, page 429, XP002754770, DOI: 10.1002/term.1586 Retrieved from the Internet: URL:http://onlinelibrary.wiley.com/doi/10. 1002/term.1586/epdf [retrieved on 2016-02-25]
- X.Guo et al.: "Bioprinting a hybrid scaffold containing human placenta derived extracellular matrix", JOURNAL OF TISSUE ENGINEERING AND REGENERATIVE MEDICINE, vol. 6 (Suppl. 1), 61.P38, 3 September 2012 (2012-09-03), 2012, page 376, XP002754771, DOI: 10.1002/term.1586 Retrieved from the Internet: URL:http://onlinelibrary.wiley.com/doi/10. 1002/term.1586/epdf [retrieved on 2016-02-25]
- SONG ET AL.: 'A Three-Dimensional Bioprinting System for Use With a Hydrogel-Based Biomaterial and Printing Parameter Characterization' ARTIFICAL ORGANS vol. 34, no. ISS. 1, 24 November 2010, pages 1044 - 1048, XP055234190

## Description

### 1. INTRODUCTION

Provided herein are methods of generating three-dimensional tissues in vivo comprising the deposition and/or administration of cells and extracellular matrix components to a subject.

### 2. BACKGROUND

Bioprinting (e.g., organ printing) is an area of research and engineering that involves printing devices, such as modified ink-jet printers, that deposit biological materials. The technology involves the rapid creation and release of liquid droplets comprising cells followed by their precise deposition on a substrate. Tissues and organs engineered using basic cellular materials by means of bioprinting represent a promising alternative to donor-derived tissues and organs used today in standard transplantation approaches.

US2003091543 discloses preparations including genetically modified cells and matrices for treating human subjects.

### 3. SUMMARY

The scope of the invention is defined by the claims. Any references in the description to methods of treatment refer to the compounds, compositions and medicaments of the present invention for use in a method for treatment of the human (or animal) body by therapy.

Provided herein are methods for forming three-dimensional tissues in vivo. In one embodiment, provided herein is a method for forming a three-dimensional tissue in vivo, comprising depositing on a surface that is in or on a subject at least one composition that comprises cells. In another embodiment, provided herein is a method for forming a three-dimensional tissue in vivo, comprising depositing on a surface that is in or on a subject at least one composition that comprises cells and at least one composition that comprises an extracellular matrix (ECM). In another embodiment, provided herein is a method for forming a three-dimensional tissue in vivo, comprising depositing on a surface that is in or on a subject at least one composition that comprises cells, at least one composition that comprises an extracellular matrix (ECM), and at least one other additional components. Cells that may be used in accordance with the methods described herein are described in Section 4.1.1, below. According to the invention, the cellular composition comprises insulin-producing cells. Tissues and Organs onto which cells, ECM, and/or other additional components can be deposited in accordance with the methods described herein are described in Section 4.1.2, below. ECM that may be used in accordance with the methods described herein is described in Section 4.1.3, below. According to the invention, the extracellular matrix (ECM) is placental extracellular matrix. Surfaces onto which cells, ECM, and/or additional components may be deposited in accordance with the methods described herein are described in Section 4.1.4, below. According to the invention the surface is in or on a human or animal subject.

In one embodiment, the cells and ECM used in the methods for forming three-dimensional tissues in vivo described herein are deposited as part of the same composition. In another embodiment, the cells and ECM used in the methods for forming three-dimensional tissues in vivo described herein are deposited as part of different compositions. In a specific embodiment, the ECM used in the methods for forming three-dimensional tissues in vivo described herein comprises flowable ECM. In another specific embodiment, the cells and ECM used in the methods for forming three-dimensional tissues in vivo described herein are deposited as part of different compositions, for example, wherein the ECM is deposited separate from, e.g., before, the deposition of the cells, and/or wherein the ECM is dehydrated prior to the deposition of the cells. In embodiments where the ECM is dehydrated, it may later be rehydrated at a desired time, e.g., at the time cells are deposited onto the surface that the ECM and cells have been deposited on.

In certain embodiments, the cells and/or ECM used in the methods for forming three-dimensional tissues in vivo described herein are deposited onto a surface in or on a subject concurrently, before, or after deposition of one or more additional components, e.g., a growth factor(s), a cross-linker(s), a polymerizable monomer(s), a polymer, a hydrogel(s), etc. In certain embodiments, the one or more additional components are bioprinted onto said surface in accordance with the methods described herein. According to the invention the cellular composition and ECM are both printed onto a surface in or on a human or animal subject.

In certain embodiments, the cells and/or ECM used in the methods for forming three-dimensional tissues in vivo described herein are deposited onto a surface in or on a subject concurrently, before, or after deposition of a biomolecule, wherein said biomolecule comprises a type of collagen, a type of fibronectin, a type of laminin, or a tissue adhesive. In certain embodiments, the biomolecule(s) is bioprinted onto said surface in accordance with the methods described herein.

In certain embodiments, the cells and/or ECM used in accordance with the methods described herein are not bioprinted onto a surface in or on a subject, e.g., the cells and ECM are not bioprinted but, rather, are applied to said surface by a method that does not comprise bioprinting. In certain embodiments, the cells and/or ECM that are not bioprinted onto a surface in or on a subject are applied to a surface that has been bioprinted, e.g., the cells and/or flowable ECM are applied to a scaffold, e.g., a synthetic scaffold, such as a synthetic matrix, that has been bioprinted in or on the subject. In a specific embodiment, the cells and/or ECM are applied to only part, e.g., one side, of the scaffold (e.g., the surface). In another specific embodiment, the cells and/or ECM are applied to all sides of the scaffold, i.e., the entire scaffold has cells and/or ECM applied to it. In another specific embodiment, the scaffold is polycaprolactone (PCL).

In certain embodiments, the cells and/or flowable ECM (as well as additional components) used in the methods for forming three-dimensional tissues in vivo described herein are printed onto said surface, e.g., the cells and ECM are bioprinted (e.g., with an inkjet printer). In certain embodiments, the cells and flowable ECM (as well as additional components) used in the methods for forming three-dimensional tissues in vivo described herein are sprayed onto said surface. In certain embodiments, the cells and/or flowable ECM (as well as additional components) used in the methods for forming three-dimensional tissues in vivo described herein are deposited onto said surface via aerosolization.

In certain embodiments, the surface onto which cells, ECM, and/or additional components may be deposited comprises an artificial surface, i.e., a surface that has been man-made. In another specific embodiment, the surface onto which cells, ECM, and/or additional components may be deposited comprises tissue or an organ (or portion thereof) of a subject (e.g., a human subject). In certain embodiments, the surface of said tissue or organ may be decellularized, e.g., treated so as to remove cells from all or part of the surface of the tissue or organ. In a specific embodiment, the surface onto which cells, ECM, and/or additional components may be deposited in accordance with the methods described herein is a surface that has been bioprinted, e.g., bioprinted in accordance with the methods described herein. In a specific embodiment, the surface is polycaprolactone (PCL).

In specific embodiments, the methods described herein are used for therapeutic purposes, e.g., the methods are used to deposit cells corresponding to a specific tissue type, or cells corresponding to multiple tissue types, onto a surface (i.e., a tissue or organ) of said subject that will benefit from the deposition of said cells. Such methods may additionally comprise the deposition of ECM (e.g., a flowable ECM) and/or additional components onto said surface of said subject.

In another aspect, provided herein are compositions comprising cells and ECM (e.g., a flowable ECM), wherein said compositions are suitable for use in the methods described herein. Also provided herein are kits comprising, in one or more containers, said compositions, as well as instructions for using said compositions in accordance with one or more of the methods described herein.

### 3.1 BRIEF DESCRIPTION OF DRAWINGS

Fig. 1 depicts scaffolds comprising polycaprolactone (PCL) that were bioprinted at various angles and in such a way that scaffolds of various pore sizes were generated.
Fig. 2 depicts multiple view of bioprinted scaffolds onto which extracellular matrix (ECM) has been applied to both sides of the scaffold and subsequently dehydrated.
Fig. 3 depicts the results of a cell proliferation assay. Placental stem cells cultured on a hybrid scaffold comprising bioprinted PCL and dehydrated ECM proliferate over an 8-day culture period.
Fig. 4 depicts the results of a cell viability assay. Placental stem cells cultured on a hybrid scaffold comprising bioprinted PCL and dehydrated ECM proliferated and remained viable over an 8-day culture period.
Fig. 5 depicts an intact three-dimensional hybrid scaffold comprising PCL, ECM, and placental stem cells, each of which were bioprinted as layers (layers of PCL and layers of ECM/cells).
Fig. 6 demonstrates that placental stem cells distribute throughout three-dimensional bioprinted scaffolds over a 7-day culture period.
Fig. 7 depicts the results of a cell viability assay. Placental stem cells bioprinted with ECM and PCL to form a three-dimensional hybrid scaffold proliferate and remain viable over a 7-day culture period.
Fig. 8 demonstrates that stem cells bioprinted with ECM and PCL to form a three-dimensional hybrid scaffold spread throughout the ECM in the hybrid scaffolds over a 7-day culture period.
Fig. 9 depicts the results of a cell proliferation assay. Placental stem cells cultured in a three-dimensional hybrid scaffold that was generated by bioprinting PCL, ECM, and placental stem cells proliferate over a 7-day culture period.
Fig. 10 depicts a bioprinted scaffold comprising PCL, placental ECM, and insulin-producing cells (β-TC-6 cells).
Fig. 11 depicts the results of a cell proliferation assay. Numbers of insulin-producing cells (β-TC-6 cells) in a bioprinted scaffold comprising PCL, placental ECM, and insulin-producing cells remained steady over a 14-day culture period.
Fig. 12 depicts levels of insulin production from bioprinted scaffolds comprising PCL, placental ECM, and insulin-producing cells (β-TC-6 cells).
Fig. 13 depicts levels of insulin production from bioprinted scaffolds comprising PCL, placental ECM, and insulin-producing cells (β-TC-6 cells) following exposure to glucose challenge (A) or under control conditions (B, C).

### 4. DETAILED DESCRIPTION

Provided herein are methods for forming three-dimensional tissues in vivo. In one embodiment, provided herein is a method for forming a three-dimensional tissue in vivo, comprising depositing on a surface that is in or on a subject at least one composition that comprises cells. In another embodiment, provided herein is a method for forming a three-dimensional tissue in vivo, comprising depositing on a surface that is in or on a subject at least one composition that comprises cells and at least one composition that comprises an extracellular matrix (ECM). According to the invention the cellular composition comprises insulin producing cells and the extracellular matrix (ECM) is placental extracellular matrix.

In another embodiment, provided herein is a method for forming a three-dimensional tissue in vivo, comprising depositing on a surface that is in or on a subject at least one composition that comprises cells, at least one composition that comprises an extracellular matrix (ECM), and at least one other additional components. According to the invention the composition is printed onto a surface in or on a human or animal subject.

Cells that may be used in accordance with the methods described herein are described in Section 4.1.1, below. Tissues and Organs onto which cells, ECM, and/or other additional components can be deposited in accordance with the methods described herein are described in Section 4.1.2, below. ECM that may be used in accordance with the methods described herein is described in Section 4.1.3, below. Surfaces onto which cells, ECM, and/or additional components may be deposited in accordance with the methods described herein are described in Section 4.1.4, below.

In one embodiment, provided herein is a method for forming a three-dimensional tissue in vivo, comprising depositing on a surface that is in or on a subject at least one composition that comprises cells. In a specific embodiment, said cells are deposited using a bioprinter. In another specific embodiment, said cells comprise a single type of cell. In another specific embodiment, said cells comprise more than one type of cell.

In another embodiment, provided herein is a method for forming a three-dimensional tissue in vivo, comprising depositing on a surface that is in or on a subject at least one composition that comprises cells and at least one composition that comprises an extracellular matrix (ECM), wherein said ECM comprises flowable ECM. In a specific embodiment, said cells and said ECM are deposited using a bioprinter. In another specific embodiment, said cells comprise a single type of cell. In another specific embodiment, said cells comprise more than one type of cell.

In another embodiment, provided herein is a method for forming a three-dimensional tissue in vivo, comprising depositing on a surface that is in or on a subject at a composition that comprises cells and extracellular matrix (ECM), wherein said ECM comprises flowable ECM. In a specific embodiment, said cells and said ECM are deposited using a bioprinter. In another specific embodiment, said cells comprise a single type of cell. In another specific embodiment, said cells comprise more than one type of cell.

In another embodiment, provided herein is a method for forming a three-dimensional tissue in vivo, comprising depositing on a surface that is in or on a subject cells, extracellular matrix (ECM), and at least one additional component. In a specific embodiment, said cells, said ECM, and said one or more additional components are deposited using a bioprinter. In another specific embodiment, said cells comprise a single type of cell. In another specific embodiment, said cells comprise more than one type of cell. In another specific embodiment, said cells, said ECM, and said one or more additional components are formulated as part of the same composition. In another specific embodiment, said cells, said ECM, and said one or more additional components are formulated as part separate compositions. In another specific embodiment, said one or more additional components is a growth factor, a polymerizable monomer, a cross-linker, a polymer, or a hydrogel.

In certain embodiments, the surface in or on a subject onto which cells, ECM, and/or additional components may be deposited in accordance with the methods described herein comprises an artificial surface, i.e., a surface that has been man-made (e.g., a prosthetic). In another specific embodiment, the surface in or on a subject onto which cells, ECM, and/or additional components may be deposited in accordance with the methods described herein comprises tissue or an organ (or portion thereof) in a subject (e.g., a human subject). In certain embodiments, the surface of said tissue or organ in a subject may be decellularized, e.g., treated so as to remove cells from all or part of the surface of the tissue or organ. In a specific embodiment, the surface onto which cells, ECM, and/or additional components may be deposited in accordance with the methods described herein is a surface that has been bioprinted, e.g., bioprinted in accordance with the methods described herein. In a specific embodiment, the surface comprises a synthetic material, e.g., a synthetic polymer. In another specific embodiment, the synthetic polymer is PCL.

In certain embodiments, the cells and ECM (e.g., a flowable ECM) are not deposited concurrently, but are deposited in layers. In a specific embodiment, a layer of cells is deposited on a surface in or on a subject, followed by the deposition of a layer of ECM on the surface in or on the subject. In another specific embodiment, a layer of ECM is deposited on a surface in or on a subject, followed by the deposition of a layer of cells on the surface in or on the subject. In certain embodiments, multiple layers of ECM can be deposited on a surface in or on a subject followed by the deposition of multiple layers of cells on the surface in or on the subject, and vice versa. Likewise, additional components that are deposited concurrently with, before, or after the deposition of cells and/or ECM may be layered among cells and ECM in accordance with the methods described herein.

In certain embodiments, the cells and ECM (e.g., a flowable ECM) are deposited such that the surface in or on a subject being deposited on is wholly covered by both cells and ECM. In other embodiments, the cells and ECM (e.g., a flowable ECM) are deposited such that the surface in or on a subject being deposited on is partially covered by both cells and ECM.

In certain embodiments, the cells and ECM (e.g., a flowable ECM) are deposited such that the surface in or on a subject being deposited on is covered by cells in specific, desired areas; and covered by ECM in specific, desired areas, wherein such specific areas may or may not overlap.

In certain embodiments, the cells and ECM (e.g., a flowable ECM) may be deposited/printed onto a surface three dimensionally. As used herein "three-dimensional printing" or "three-dimensional deposition" refers to the process of printing/depositing such that, e.g., the print heads of a bioprinter move below, above, and around a three-dimensional surface (e.g., an organ or bone in a subject), e.g., the printer heads are mechanically controlled so as to rotate along a specified path. As used herein, three-dimensional printing and three-dimensional deposition is in contrast to standard methods of bioprinting that are known in the art, where the printing is performed by starting to build tissue on a flat/planar/two-dimensional surface.

### 4.1 BIOPRINTING

"Bioprinting," as used herein, generally refers to the deposition of living cells, as well as other components (e.g., a flowable ECM; synthetic matrices) onto a surface using standard or modified printing technology, e.g., ink jet printing technology. Basic methods of depositing cells onto surfaces, and of bioprinting cells, including cells in combination with hydrogels, are described in Warren et al. US 6,986,739, Boland et al. US 7,051,654, Yoo et al. US 2009/0208466 and Xu et al. US 2009/0208577. Additionally, bioprinters suitable for the methods described herein are commercially available, e.g., the 3D-Bioplotter™ from Envisiontec GmbH (Gladbeck, Germany); and the NovoGen MMX Bioprinter™ from Organovo (San Diego, CA).

The bioprinter used in the methods described herein may include mechanisms and/or software that enables control of the temperature, humidity, shear force, speed of printing, and/or firing frequency, by modifications of, e.g., the printer driver software and/or the physical makeup of the printer. In certain embodiments, the bioprinter software and/or hardware preferably may be constructed and/or set to maintain a cell temperature of about 37°C during printing.

In certain embodiments, the inkjet printing device may include a two-dimensional or three-dimensional printer. In certain embodiments, the bioprinter comprises a DC solenoid inkjet valve, one or more reservoir for containing one or more types of cells, e.g., cells in a flowable composition, and/or ECM (e.g., a flowable ECM) prior to printing, e.g., connected to the inkjet valve. The bioprinter may have 1, 2, 3, 4, 5, 6, 7, 8, 9, 10 or more reservoirs, e.g., one for each cell type or each ECM used to construct the tissues and organs described herein. The cells may be delivered from the reservoir to the inkjet valve by air pressure, mechanical pressure, or by other means. Typically, the bioprinter, e.g., the print heads in the bioprinter, is/are computer-controlled such that the one or more cell types, and said ECM, are deposited in a predetermined pattern. Said predetermined pattern can be a pattern that recreates or recapitulates the natural arrangement of said one or more types of cells in an organ or tissue from which the cells are derived or obtained, or a pattern that is different from the natural arrangement of said one or more types of cells.

In certain embodiments, the bioprinter used in the methods provided herein may be a thermal bubble inkjet printer, see, e.g., Niklasen et al. US 6,537,567, or a piezoelectric crystal vibration print head, e.g., using frequencies up to 30 kHz and power sources ranging from 12 to 100 Watts. Bioprinter print head nozzles, in some embodiments, are each independently between 0.05 and 200 micrometers in diameter, or between 0.5 and 100 micrometers in diameter, or between 10 and 70 micrometers in diameter, or between 20 and 60 micrometers in diameter. In further embodiments, the nozzles are each independently about 40 or 50 micrometers in diameter. Multiple nozzles with the same or different diameters may be used. In some embodiments the nozzles have a circular opening; in other embodiments, other suitable shapes may be used, e.g., oval, square, rectangle, etc., without departing from the spirit of the invention.

In certain embodiments, the bioprinter used in accordance with the methods described herein comprises a plurality of print heads and/or a plurality of print jets, wherein said plurality of print heads or print jets may, in certain embodiments, be separately controllable. In certain embodiments, each of said print heads or print jets operates independently from the remaining said print heads or print jets. In certain embodiments, at least one of said plurality of print heads or plurality of print jets may print compositions comprising cells, and at least one of said plurality of print heads or plurality of print jets may print compositions comprising ECM. In certain embodiments, at least one of said plurality of print heads or plurality of print jets may print compositions comprising cells, at least one of said plurality of print heads or plurality of print jets may print compositions comprising ECM, and at least one of said plurality of print heads or plurality of print jets may print compositions comprising an additional component.

In certain embodiments, one or more print heads or print jets of a bioprinter used in accordance with the methods described herein may be modified so that it is suitable for printing on certain surface. For example, a print head or print jet may be modified by attaching it to a certain surgical instrument, e.g., a laparoscope. In accordance with such embodiments, the surgical instrument may be fitted with one or more other components that aid in the printing procedure, e.g., a camera.

In certain embodiments, an anatomical image of the tissue or organ to be printed on may be constructed using software, e.g., a computer-aided design (CAD) software program. In accordance with such embodiments, programs can be generated that allow for three-dimensional printing on a three-dimensional surface that is representative of the structure of the tissue or organ to be printed on. For example, if it is desired to print on a bone, an anatomical image of the bone may be constructed and a program may be generated that directs the printer heads of the bioprinter to rotate around the three-dimensional bone inside the subject surface during printing.

In certain embodiments, the surface of the tissue or organ to be printed on is scanned in or on said the subject so as to form a surface map, and said surface map is used to guide the depositing of the cells, ECM, and/or any additional components to be printed. Such scanning may comprise, without limitation, the use of a laser, electron beam, magnetic resonance imaging, microwave, or computed tomography. The scan of said surface may comprise a resolution of least 1000, 100, 10, 1, or 0.1 microns.

In certain embodiments, the methods of bioprinting provided herein comprise the delivery/deposition of individual droplets of cells (e.g., compositions comprising single cells or compositions comprising multiple cells) and flowable extracellular matrix (ECM) on a surface in or on a subject.

In certain embodiments, the methods of bioprinting provided herein comprise the deposition of a single cell type and flowable ECM on a surface in or on a subject. Exemplary cell types that can be used in accordance with such methods are provided in Section 4.1.1, below. ECM, including flowable ECM, is described in Section 4.1.3, below.

In other embodiments, the methods of bioprinting provided herein comprise the deposition of multiple (e.g., two, three, four, five or more) cell types and flowable ECM on a surface in or on a subject. In a specific embodiment, the multiple cell types are deposited as part of the same composition, i.e., the source of the cells is a single composition that comprises the multiple cell types. In another specific embodiment, the multiple cell types are deposited as part of different compositions, i.e., the source of the cells are distinct compositions that comprise the multiple cell types. In another specific embodiment, a portion of the multiple cell types are deposited as part of one composition (e.g., two or more cell types are in a single composition) and another portion of the multiple types are deposited as a different composition (e.g., one or more cell types are in a single composition). Exemplary cell types that can be used in accordance with such methods are provided in Section 4.1.2, below.

In a specific embodiment, the cells to be deposited and the flowable ECM are deposited on a surface in or on a subject together (e.g., simultaneously) as part of the same composition. In another specific embodiment, the cells to be deposited and the flowable ECM are deposited on a surface in or on a subject together as part of different compositions. In another specific embodiment, the cells to be deposited and the flowable ECM are deposited on a surface in or on a subject separately (e.g., at different times).

In certain embodiments, the cells and flowable ECM are deposited on a surface in or on a subject with one or more additional components. In one embodiment, the one or more additional components are formulated in the same composition as the cells. In another embodiment, the one or more additional components are formulated in the same composition as the ECM. In another embodiment, the one or more additional components are formulated in the same composition as the cells and the ECM (i.e., a single composition comprises the cells, the flowable ECM, and the one or more additional components). In another embodiment, the one or more additional components are formulated in a composition that is separate from the compositions comprising the cells and/or ECM, and is deposited concurrently with, before, or after the deposition of the cells and/or ECM on a surface in or on a subject. In a specific embodiment, the one or more additional components promote the survival, differentiation, proliferation, etc. of the cell(s). In another specific embodiment, the one or more additional components comprise a cross-linker (see Section 4.1.3.2). In another specific embodiment, the one or more additional components comprise a hydrogel. In another specific embodiment, the one or more additional components comprise a synthetic polymer.

Those of skill in the art will recognize that the cells and flowable ECM, as well as any additional components used in accordance with the methods described herein, may be printed from separate nozzles of a printer, or through the same nozzle of a printer in a common composition, depending upon the particular tissue or organ being formed. It also will be recognized by those of skill in the art that the printing may be simultaneous or sequential, or any combination thereof and that some of the components (e.g., cells, flowable ECM, or additional components) may be printed in the form of a first pattern and some of the components may be printed in the form of a second pattern, and so on. The particular combination and manner of printing will depend upon the particular tissue or organ in a subject that is being printed on.

In certain embodiments, the cells, ECM, and/or any other materials (e.g., synthetic matrices, e.g., PCL) may be bioprinted in a specified pattern so as to yield a desired result. For example, bioprinted materials (e.g., cells, ECM, matrices, and other components described herein) may be bioprinted in layers at varying angles so as to generate specific desirable patterns, such as three-dimensional structures having specific pore sizes. In a specific embodiment, bioprinted materials (e.g., cells, ECM, matrices, and other components described herein) are printed in a criss-cross fashion so as to generate a bioprinted structure with pores of desired sizes that appear box-like. In another specific embodiment, bioprinted materials (e.g., cells, ECM, matrices, and other components described herein) are printed angles, so as to generate pores of desired sizes that appear triangular or diamond-like. For example, bioprinted materials (e.g., cells, ECM, matrices, and other components described herein) at angles of specific degrees, e.g., 30 degree angles, 45 degree angles, 60 degree angles, in order to generate desired patterns. In accordance with such methods of printing, bioprinted structures having desirable qualities, e.g., the ability to foster cellular growth and proliferation, can be generated. See Example 1, below. In a specific embodiment, matrices, e.g., synthetic matrices are bioprinted in specific patterns that are conducive to supporting the growth and proliferation of cells on said bioprinted matrices. In specific embodiments, the synthetic matrix is PCL.

### 4.1.1 CELLS

Any type of cell known in the art can be used in accordance with the methods described herein, including prokaryotic and eukaryotic cells.

The cells used in accordance with the methods described herein may be syngeneic (i.e., genetically identical or closely related to the cells of the recipient subject, so as to minimize tissue transplant rejection), allogeneic (i.e., from a non-genetically identical member of the same species of the recipient subject) or xenogeneic (i.e., from a member of a different species than the recipient subject). Syngeneic cells include those that are autogeneic (i.e., from the recipient subject) and isogeneic (i.e., from a genetically identical but different subject, e.g., from an identical twin). Cells may be obtained from, e.g., a donor (either living or cadaveric) or derived from an established cell strain or cell line. For example, cells may be harvested from a donor (e.g., a potential recipient) using standard biopsy techniques known in the art.

In certain embodiments, the cells used in accordance with the methods described herein are contained within a flowable physiologically-acceptable composition, e.g., water, buffer solutions (e.g., phosphate buffer solution, citrate buffer solution, etc.), liquid media (e.g., 0.9N saline solution, Kreb's solution, modified Kreb's solution, Eagle's medium, modified Eagle's medium (MEM), Dulbecco's Modified Eagle's Medium (DMEM), Hank's Balanced Salts, etc.), and the like.

In certain embodiments, the cells used in accordance with the methods described herein may comprise primary cells that have been isolated from a tissue or organ, using one or more art-known proteases, e.g., collagenase, dispase, trypsin, LIBERASE, or the like. Organ tissue may be physically dispersed prior to, during, or after treatment of the tissue with a protease, e.g., by dicing, macerating, filtering, or the like. Cells may be cultured using standard, art-known cell culture techniques prior to use of the cells in the methods described herein, e.g., in order to produce homogeneous or substantially homogeneous cell populations, to select for particular cell types, or the like.

In one embodiment, the cell type(s) used in the methods described herein comprises stem cells. A non-limiting list of stem cells that can be used in accordance with the methods described herein includes: embryonic stem cells, induced pluripotent stem cells, mesenchymal stem cells, bone marrow-derived mesenchymal stem cells (BM-MSCs), tissue plastic-adherent placental stem cells (PDACs), umbilical cord stem cells, amniotic fluid stem cells, amnion derived adherent cells (AMDACs), osteogenic placental adherent cells (OPACs), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, or side population stem cells.

In a specific embodiment, the methods described herein comprise the use of placental stem cells (e.g., the placental stem cells described in US 7,468,276 and US 8,057,788). In another specific embodiment, said placental stem cells are PDACs®. In one embodiment, said PDACs are CD34-, CD10+, CD105+, and CD200+. In another embodiment, said PDACs are CD34-, CD10+, CD105+, and CD200+ and additionally are CD45-, CD80-, CD86-, and/or CD90+.

In another specific embodiment, the methods described herein comprise the use of AMDACs (e.g., the AMDACs described in international application publication no. WO10/059828). In one embodiment, said AMDACs are Oct4-. In another embodiment, said AMDACs are CD49f+. In another embodiment, said AMDACs are Oct4- and CD49f+.

In another specific embodiment, the methods described herein comprise the use of PDACs and AMDACs.

In another specific embodiment, the methods described herein comprise the use of BM-MSCs.

In another embodiment, the cell type(s) used in the methods described herein comprise differentiated cells. In a specific embodiment, the differentiated cell(s) used in accordance with the methods described herein comprise endothelial cells, epithelial cells, dermal cells, endodermal cells, mesodermal cells, fibroblasts, osteocytes, chondrocytes, natural killer cells, dendritic cells, hepatic cells, pancreatic cells, and/or stromal cells. According to the invention the cells are insulin-producing cells, e.g., pancreatic cells (e.g., islet cells) or an insulin-producing cell line, e.g., β-TC-6 cells.

In another specific embodiment, the differentiated cell(s) used in accordance with the methods described herein comprise salivary gland mucous cells, salivary gland serous cells, von Ebner's gland cells, mammary gland cells, lacrimal gland cells, ceruminous gland cells, eccrine sweat gland dark cells, eccrine sweat gland clear cells, apocrine sweat gland cells, gland of Moll cells, sebaceous gland cells. bowman's gland cells, Brunner's gland cells, seminal vesicle cells, prostate gland cells, bulbourethral gland cells, Bartholin's gland cells, gland of Littre cells, uterus endometrium cells, isolated goblet cells, stomach lining mucous cells, gastric gland zymogenic cells, gastric gland oxyntic cells, pancreatic acinar cells, paneth cells, type II pneumocytes, and/or clara cells.

In another specific embodiment, the differentiated cell(s) used in accordance with the methods described herein comprise somatotropes, lactotropes, thyrotropes, gonadotropes, corticotropes, intermediate pituitary cells, magnocellular neurosecretory cells, gut cells, respiratory tract cells, thyroid epithelial cells, parafollicular cells, parathyroid gland cells, parathyroid chief cell, oxyphil cell, adrenal gland cells, chromaffin cells, Leydig cells, theca interna cells, corpus luteum cells, granulosa lutein cells, theca lutein cells, juxtaglomerular cell, macula densa cells, peripolar cells, and/or mesangial cells.

In another specific embodiment, the differentiated cell(s) used in accordance with the methods described herein comprise blood vessel and lymphatic vascular endothelial fenestrated cells, blood vessel and lymphatic vascular endothelial continuous cells, blood vessel and lymphatic vascular endothelial splenic cells, synovial cells, serosal cell (lining peritoneal, pleural, and pericardial cavities), squamous cells, columnar cells, dark cells, vestibular membrane cell (lining endolymphatic space of ear), stria vascularis basal cells, stria vascularis marginal cell (lining endolymphatic space of ear), cells of Claudius, cells of Boettcher, choroid plexus cells, pia-arachnoid squamous cells, pigmented ciliary epithelium cells, nonpigmented ciliary epithelium cells, corneal endothelial cells, peg cells, respiratory tract ciliated cells, oviduct ciliated cell, uterine endometrial ciliated cells, rete testis ciliated cells, ductulus efferens ciliated cells, and/or ciliated ependymal cells.

In another specific embodiment, the differentiated cell(s) used in accordance with the methods described herein comprise epidermal keratinocytes, epidermal basal cells, keratinocyte of fingernails and toenails, nail bed basal cells, medullary hair shaft cells, cortical hair shaft cells, cuticular hair shaft cells, cuticular hair root sheath cells, hair root sheath cells of Huxley's layer, hair root sheath cells of Henle's layer, external hair root sheath cells, hair matrix cells, surface epithelial cells of stratified squamous epithelium, basal cell of epithelia, and/or urinary epithelium cells.

In another specific embodiment, the differentiated cell(s) used in accordance with the methods described herein comprise auditory inner hair cells of organ of Corti, auditory outer hair cells of organ of Corti, inner pillar cells of organ of Corti, outer pillar cells of organ of Corti, inner phalangeal cells of organ of Corti, outer phalangeal cells of organ of Corti, border cells of organ of Corti, Hensen cells of organ of Corti, vestibular apparatus supporting cells, taste bud supporting cells, olfactory epithelium supporting cells, Schwann cells, satellite cells, enteric glial cells, basal cells of olfactory epithelium, cold-sensitive primary sensory neurons, heat-sensitive primary sensory neurons, Merkel cells of epidermis, olfactory receptor neurons, pain-sensitive primary sensory neurons, photoreceptor rod cells, photoreceptor blue-sensitive cone cells, photoreceptor green-sensitive cone cells, photoreceptor red-sensitive cone cells, proprioceptive primary sensory neurons, touch-sensitive primary sensory neurons, type I carotid body cells, type II carotid body cell (blood pH sensor), type I hair cell of vestibular apparatus of ear (acceleration and gravity), type II hair cells of vestibular apparatus of ear, type I taste bud cells, cholinergic neural cells, adrenergic neural cells, and/or peptidergic neural cells.

In another specific embodiment, the differentiated cell(s) used in accordance with the methods described herein comprise astrocytes, neurons, oligodendrocytes, spindle neurons, anterior lens epithelial cells, crystallin-containing lens fiber cells, hepatocytes, adipocytes, white fat cells, brown fat cells, liver lipocytes, kidney glomerulus parietal cells, kidney glomerulus podocytes, kidney proximal tubule brush border cells, loop of Henle thin segment cells, kidney distal tubule cells, kidney collecting duct cells, type I pneumocytes, pancreatic duct cells, nonstriated duct cells, duct cells, intestinal brush border cells, exocrine gland striated duct cells, gall bladder epithelial cells, ductulus efferens nonciliated cells, epididymal principal cells, and/or epididymal basal cells.

In another specific embodiment, the differentiated cell(s) used in accordance with the methods described herein comprise ameloblast epithelial cells, planum semilunatum epithelial cells, organ of Corti interdental epithelial cells, loose connective tissue fibroblasts, corneal keratocytes, tendon fibroblasts, bone marrow reticular tissue fibroblasts, nonepithelial fibroblasts, pericytes, nucleus pulposus cells, cementoblast/cementocytes, odontoblasts, odontocytes, hyaline cartilage chondrocytes, fibrocartilage chondrocytes, elastic cartilage chondrocytes, osteoblasts, osteocytes, osteoclasts, osteoprogenitor cells, hyalocytes, stellate cells (ear), hepatic stellate cells (Ito cells), pancreatic stelle cells, red skeletal muscle cells, white skeletal muscle cells, intermediate skeletal muscle cells, nuclear bag cells of muscle spindle, nuclear chain cells of muscle spindle, satellite cells, ordinary heart muscle cells, nodal heart muscle cells, Purkinje fiber cells, mooth muscle cells, myoepithelial cells of iris, and/or myoepithelial cells of exocrine glands.

In another specific embodiment, the differentiated cell(s) used in accordance with the methods described herein comprise reticulocytes, megakaryocytes, monocytes, connective tissue macrophages. epidermal Langerhans cells, dendritic cells, microglial cells, neutrophils, eosinophils, basophils, mast cell, helper T cells, suppressor T cells, cytotoxic T cell, natural Killer T cells, B cells, natural killer cells, melanocytes, retinal pigmented epithelial cells, Sertoli cells, thymus epithelial cell, and/or interstitial kidney cells.

The cells used in accordance with the methods described herein can be formulated in compositions. In certain embodiments, the cells used in accordance with the methods described herein are formulated in compositions that comprise only a single cell type, i.e., the population of cells in the composition is homogeneous. In other embodiments, the cells used in accordance with the methods described herein are formulated in compositions that comprise more than one cell type, i.e., the population of cells in the composition is heterogeneous.

In certain embodiments, the cells used in accordance with the methods described herein are formulated in compositions that additionally comprise flowable ECM (see Section 4.1.3). Alternatively, said flowable ECM may be deposited as part of a separate composition in accordance with the methods described herein concurrently with, before, or after the deposition of said cells. In certain embodiments, the cells used in accordance with the methods described herein are formulated in compositions that additionally comprise one or more synthetic monomers or polymers. Alternatively, said synthetic monomers or polymers may be deposited as part of a separate composition in accordance with the methods described herein concurrently with, before, or after the deposition of said cells. In certain embodiments, the cells used in accordance with the methods described herein are formulated in compositions that additionally comprise flowable ECM and one or more synthetic monomers or polymers. In certain embodiments, the cells used in accordance with the methods described herein are formulated in compositions that additionally comprise a cross-linking agent. Alternatively, said cross-linking agent may be deposited as part of a separate composition in accordance with the methods described herein concurrently with, before, or after the deposition of said cells.

In certain embodiments, the cells used in accordance with the methods described herein are formulated in compositions that additionally comprise one or more additional components, e.g., components that promote the survival, differentiation, proliferation, etc. of the cell(s). Such components may include, without limitation, nutrients, salts, sugars, survival factors, and growth factors. Exemplary growth factors that may be used in accordance with the methods described herein include, without limitation, insulin-like growth factor (e.g., IGF-1), transforming growth factor-beta (TGF-beta), bone-morphogenetic protein, fibroblast growth factor, platelet derived growth factor (PDGF), vascular endothelial growth factor (VEGF), connective tissue growth factor (CTGF), basic fibroblast growth factor (bFGF), epidermal growth factor, fibroblast growth factor (FGF) (numbers 1, 2 and 3), osteopontin, bone morphogenetic protein-2, growth hormones such as somatotropin, cellular attractants and attachment agents, etc., and mixtures thereof. Alternatively, said one or more additional components that promote the survival, differentiation, proliferation, etc. of the cell(s) may be deposited as part of a separate composition in accordance with the methods described herein concurrently with, before, or after the deposition of said cells.

In certain embodiments, the cells used in accordance with the methods described herein are primary culture cells, e.g., cells that have been cultured in vitro. Such primary cells can be passaged once or multiple times, e.g. twice, three times, four times, five times, six times, seven times, eight times, nine times, ten times, or more than ten times. In a specific embodiment, said primary cells have been passaged no more than six times. In another specific embodiment, said primary cells are derived from the subject to be printed in or on.

In certain embodiments, the cells used in accordance with the methods described herein are genetically engineered to produce a protein(s) or polypeptide(s) that is not naturally produced by the cell, or have been genetically engineered to produce a protein(s) or polypeptide(s) in an amount that is greater than that naturally produced by the cell. In a specific embodiment, such cells comprise or consist of differentiated cells. In another specific embodiment, said cells comprise a plasmid that directs the production of said protein or polypeptide. Such cells may be cultured in such a manner that the amount of protein or polypeptide can be controlled and/or optimized. For example, said cells may be engineered so that approximately 1 x 10⁶ of said cells produces about or at least 0.01 to 0.1, 0.1 to 1.0, 1.0 to 10.0, or 10.0 to 100.0 µM of said protein or polypeptide in an in vitro culture in growth medium over approximately 24 hours.

In a specific embodiment, the cells used in accordance with the methods described herein are genetically engineered to produce a cytokine or a peptide comprising an active part thereof. Exemplary cytokines that may be produced by such engineered cells include, without limitation, adrenomedullin (AM), angiopoietin (Ang), bone morphogenetic protein (BMP), brain-derived neurotrophic factor (BDNF), epidermal growth factor (EGF), erythropoietin (Epo), fibroblast growth factor (FGF), glial cell line-derived neurotrophic factor (GNDF), granulocyte colony stimulating factor (G-CSF), granulocyte-macrophage colony stimulating factor (GM-CSF), growth differentiation factor (GDF-9), hepatocyte growth factor (HGF), hepatoma derived growth factor (HDGF), insulin-like growth factor (IGF), migration-stimulating factor, myostatin (GDF-8), myelomonocytic growth factor (MGF), nerve growth factor (NGF), placental growth factor (P1GF), platelet-derived growth factor (PDGF), thrombopoietin (Tpo), transforming growth factor alpha (TGF-α), TGF-β, tumor necrosis factor alpha (TNF-α), vascular endothelial growth factor (VEGF), or a Wnt protein.

In another specific embodiment, the cells used in accordance with the methods described herein are genetically engineered to produce a soluble receptor for a protein or polypeptide, e.g., a soluble receptor for a cytokine. Exemplary soluble receptors that may be produced by such cells include, without limitation, soluble receptors for AM, Ang, BMP, BDNF, EGF, Epo, FGF, GNDF, G-CSF, GM-CSF, GDF-9, HGF, HDGF, IGF, migration-stimulating factor, GDF-8, MGF, NGF, P1GF, PDGF, Tpo, TGF-α, TGF-β, TNF-α, VEGF, and Wnt protein.

In another specific embodiment, the cells used in accordance with the methods described herein are genetically engineered to produce an interleukin. Exemplary interleukins that may be produced by such cells include, without limitation, interleukin-1 alpha (IL-1α), IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, both IL-12 alpha and beta subunits, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-23 p19 subunit and IL-23 p40 subunit together, IL-24, IL-25, IL-26, IL-27B, IL-27-p28, IL-27B and IL-27-p28 together, IL-28A, IL-28B, IL-29, IL-30, IL-31, IL-32, IL-33, IL-34, IL-35, IL-36α, IL-36β, and IL-36γ.

In another specific embodiment, the cells used in accordance with the methods described herein are genetically engineered to produce a soluble receptor for an interleukin. Exemplary soluble receptors for interleukins that may be produced by such cells include, without limitation, soluble receptors for IL-1α, IL-1β, IL-1F1, IL-1F2, IL-1F3, IL-1F4, IL-1F5, IL-1F6, IL-1F7, IL-1F8, IL-1F9, IL-2, IL-3, IL-4, IL-5, IL-6, IL-7, IL-8, IL-9, IL-10, IL-11, IL-12 35 kDa alpha subunit, IL-12 40 kDa beta subunit, IL-13, IL-14, IL-15, IL-16, IL-17A, IL-17B, IL-17C, IL-17D, IL-17E, IL-17F isoform 1, IL-17F isoform 2, IL-18, IL-19, IL-20, IL-21, IL-22, IL-23 p19 subunit, IL-23 p40 subunit, IL-24, IL-25, IL-26, IL-27B, IL-27-p28, IL-28A, IL-28B, IL-29, IL-30, IL-31, IL-32, IL-33, IL-34, IL-35, IL-36α, IL-36β, and IL-36γ.

In another specific embodiment, the cells used in accordance with the methods described herein are genetically engineered to produce an interferon. Exemplary interferons that may be produced by such cells include, without limitation, IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω, and IFN-v.

In another specific embodiment, the cells used in accordance with the methods described herein are genetically engineered to produce a soluble receptor for an interferon. Exemplary soluble receptors for interferons that may be produced by such cells include, without limitation, soluble receptors for IFN-α, IFN-β, IFN-γ, IFN-λ1, IFN-λ2, IFN-λ3, IFN-K, IFN-ε, IFN-κ, IFN-τ, IFN-δ, IFN-ζ, IFN-ω, or IFN-v.

Also according to the invention, the cells used in accordance with the methods described herein are genetically engineered to produce insulin or proinsulin. In another specific embodiment, the cells used in accordance with the methods described herein are genetically engineered to produce a receptor for insulin. In certain embodiments, said cells genetically engineered to produce insulin or proinsulin, and/or a receptor for insulin, are additionally genetically engineered to produce one or more of prohormone convertase 1, prohormone convertase 2, or carboxypeptidase E.

In another specific embodiment, the cells used in accordance with the methods described herein are genetically engineered to produce leptin. In another specific embodiment, the cells used in accordance with the methods described herein are genetically engineered to produce erythropoietin (EPO). In another specific embodiment, the cells used in accordance with the methods described herein are genetically engineered to produce thrombopoietin.

In another specific embodiment, the cells used in accordance with the methods described herein are genetically engineered to produce tyrosine 3-monooxygenase, a protein capable of producing L-DOPA from 1-tyrosine. In certain embodiments, said cells are further engineered to express aromatic L-amino acid decarboxylase, which produces dopamine from L-DOPA.

In another specific embodiment, the cells used in accordance with the methods described herein are genetically engineered to produce a hormone or prohormone. Exemplary hormones that may be produced by such cells include, without limitation, antimullerian hormone (AMH), adiponectin (Acrp30), adrenocorticotropic hormone (ACTH), angiotensin (AGT), angiotensinogen (AGT), antidiuretic hormone (ADH), vasopressin, atrial-natriuretic peptide (ANP), calcitonin (CT), cholecystokinin (CCK), corticotrophin-releasing hormone (CRH), erythropoietin (Epo), follicle-stimulating hormone (FSH), testosterone, estrogen, gastrin (GRP), ghrelin, glucagon (GCG), gonadotropin-releasing hormone (GnRH), growth hormone (GH), growth hormone releasing hormone (GHRH), human chorionic gonadotropin (hCG), human placental lactogen (HPL), inhibin, leutinizing hormone (LH), melanocyte stimulating hormone (MSH), orexin, oxytocin (OXT), parathyroid hormone (PTH), prolactin (PRL), relaxin (RLN), secretin (SCT), somatostatin (SRIF), thrombopoietin (Tpo), thyroid-stimulating hormone (Tsh), and thyrotropin-releasing hormone (TRH).

In another specific embodiment, the cells used in accordance with the methods described herein are genetically engineered to produce cytochrome P450 side chain cleavage enzyme (P450SCC).

In another specific embodiment, the cells used in accordance with the methods described herein are genetically engineered to produce low density lipoprotein receptor (LDLR).

In another specific embodiment, the cells used in accordance with the methods described herein are genetically engineered to produce polycystin-1 (PKD1), PKD-2 or PKD3.

In another specific embodiment, the cells used in accordance with the methods described herein are genetically engineered to produce phenylalanine hydroxylase.

In certain embodiments, the cells used in accordance with the methods described herein are formulated in compositions that additionally comprise a polymerizable monomer(s). In such embodiments, for example, a polymerization catalyst may be added immediately prior to bioprinting, such that once the cells are printed, the monomer polymerizes, forming a gel that traps and/or physically supports the cells. For example, the composition comprising the cells can comprise acrylamide monomers, whereupon TEMED and Ammonium persulfate, or riboflavin, are added to the composition immediately prior to bioprinting. Upon deposition of the cells in the composition onto a surface, the acrylamide polymerizes, sequestering and supporting the cells.

In certain embodiments, the cells used in accordance with the methods described herein are formulated in compositions that additionally comprise adhesives. In a specific embodiment, the cells used in accordance with the methods described herein are formulated in compositions that additionally comprise soft tissue adhesives including, without limitation, cyanoacrylate esters, fibrin sealant, and/or gelatin-resorcinol-formaldehyde glues. In another specific embodiment, the cells used in accordance with the methods described herein are formulated in compositions that additionally comprise arginine-glycine-aspartic acid (RGD) ligands, extracellular proteins, and/or extracellular protein analogs.

In certain embodiments, the cells used in accordance with the methods described herein are formulated in compositions such that the cells can be deposited on a surface in or on a subject as single cells (i.e., the cells are deposited one cell at a time).

In certain embodiments, the cells used in accordance with the methods described herein are formulated in compositions such that the cells can be deposited on a surface in or on a subject as aggregates that comprise multiple cells. Such aggregates may comprise cells of single type, or may comprise multiple cell types, e.g., two, three, four, five or more cell types.

In certain embodiments, the cells used in accordance with the methods described herein are formulated in compositions such that the cells form a tissue as part of the composition, wherein said tissue can be deposited on a surface in or on a subject using the methods described herein. Such tissues may comprise cells of single type, or may comprise multiple cell types, e.g., two, three, four, five or more cell types.

In certain embodiments, the cells used in accordance with the methods described herein are deposited onto a surface in or on a subject as individual droplets of cells and/or compositions having small volumes, e.g., from 0.5 to 500 picoliters per droplet. In various embodiments, the volume of cells, or composition comprising the cells, is about 0.5, 1, 2, 3, 4, 5, 6, 7, 8, 9, 10, 15, 20, 25, 20, 35, 40, 45, 50, 55, 60, 65, 70, 75, 80, 85, 90, 95 or 100 picoliters, or between about 1 to 90 picoliters, about 5 to 85 picoliters, about 10 to 75 picoliters, about 15 to 70 picoliters, about 20 to 65 picoliters, or about 25 to about 60 picoliters.

### 4.1.2 TISSUES AND ORGANS

Provided herein are methods of bioprinting on tissues and organs in or on a subject using one or more of the methods provided herein. Also provided herein are methods of bioprinting new tissue in or on a subject. Also provided herein are methods of bioprinting tissue in or on existing organs in a subject, as well as the generation of new organs in a subject.

Any type of tissue known in the art can be printed in or on a subject using the methods described herein. In certain embodiments, the tissue printed in or on a subject comprises a single cell type. In other embodiments, the tissue printed in or on a subject multiple cell types. In certain embodiments, the tissue printed in or on a subject comprises more than one type of tissue.

In certain embodiments, the methods described herein comprise deposition of cells, ECM, and/or other components on a surface in or on a subject, wherein said surface comprises tissue from a subject. In certain embodiments, the methods described herein comprise deposition of cells, ECM, and/or other components on a surface in or on a subject, wherein said surface comprises an organ of the subject.

In a specific embodiment, the cells printed in or on a subject comprise connective tissue cells. In certain embodiments, said connective tissue cells are printed on connective tissue of said subject (i.e., connective tissue in or on the subject).

In another specific embodiment, the cells printed in or on a subject comprise muscle tissue cells. In certain embodiments, said muscle tissue cells are printed on muscle tissue of said subject (i.e., muscle tissue in or on the subject). The muscle tissue can comprise visceral (smooth) muscle tissue, skeletal muscle tissue, or cardiac muscle tissue.

In another specific embodiment, the cells printed in or on a subject comprise neural tissue cells. In certain embodiments, said neural tissue cells are printed on neural tissue of said subject (i.e., neural tissue in or on the subject). The neural tissue can comprise central nervous system tissue (e.g., brain tissue or spinal cord tissue) or peripheral nervous system tissue (e.g., cranial nerves and spinal nerves).

In another specific embodiment, the cells printed in or on a subject comprise epithelial tissue cells. In certain embodiments, said epithelial tissue cells are printed on epithelial tissue of said subject (i.e., epithelial tissue in or on the subject). The epithelial tissue can comprise endothelium.

In certain embodiments, the cells, ECM, and/or other components printed in or on a subject are printed on an organ of the subject. The cells, ECM, and/or other components can be printed in or on an organ of a subject that is associated with any of the known mammalian organ systems, i.e., the digestive system, circulatory system, endocrine system, excretory system, immune system, integumentary system, muscular system, nervous system, reproductive system, respiratory system, and/or skeletal system. Exemplary organs that can be generated or formed in accordance with the methods described herein include, without limitation, lungs, liver, heart, brain, kidney, skin, bone, stomach, pancreas, bladder, gall bladder, small intestine, large intestine, prostate, testes, ovaries, spinal cord, pharynx, larynx, trachea, bronchi, diaphragm, ureter, urethra, esophagus, colon, thymus, and spleen. In a specific embodiment, a pancreas or portion thereof is generated or formed in accordance with the methods described herein.

In a specific embodiment, the cells, ECM, and/or other components printed in or on a subject are printed on bone. In another specific embodiment, the cells, ECM, and/or other components printed in or on a subject are printed on skin. In another specific embodiment, the cells, ECM, and/or other components printed in or on a subject are not printed on skin. In another specific embodiment, the cells, ECM, and/or other components printed in or on a subject are printed on lung tissue, or a lung or portion thereof. In another specific embodiment, the cells, ECM, and/or other components printed in or on a subject are printed on liver tissue, or a liver or portion thereof. In another specific embodiment, the cells, ECM, and/or other components printed in or on a subject are printed on neural tissue, or a nerve or portion thereof.

### 4.1.3 EXTRACELLULAR MATRIX (ECM)

The methods described herein comprise the deposition of cells (e.g., compositions comprising single cells and/or compositions comprising multiple cells) and extracellular matrix (ECM), including flowable ECM, on a surface in or on a subject. The ECM can be derived from any known source of ECM, and can be made flowable using any method known in the art. In specific embodiments, the ECM comprises flowable ECM. The ECM can be made flowable using, e.g., the methods described in Section 4.1.3.1, below. In certain embodiments, the ECM can be cross-linked using, e.g., using the methods described in Section 4.1.3.2, below.

The ECM (e.g., a flowable ECM) used in accordance with the methods described herein can be formulated as part of a composition for use in accordance with the methods provided herein.

In certain embodiments, the ECM used in accordance with the methods described herein comprises mammalian ECM, plant ECM, molluscan ECM, and/or piscine ECM.

In a specific embodiment, the ECM used in accordance with the methods described herein comprises mammalian ECM. According to the invention, the ECM used in accordance with the methods described herein comprises mammalian ECM, wherein said mammalian ECM is derived from a placenta (e.g., a human placenta). In another specific embodiment, said placental-derived ECM comprises telopeptide collagen.

In another specific embodiment, said placental-derived ECM comprises base-treated and/or detergent treated Type I telopeptide placental collagen that has not been chemically modified or contacted with a protease, wherein said ECM comprises less than 5% fibronectin or less than 5% laminin by weight; between 25% and 92% Type I collagen by weight; and 2% to 50% Type III collagen or 2% to 50% type IV collagen by weight.

In another specific embodiment, said placental-derived ECM comprises base-treated, detergent treated Type I telopeptide placental collagen that has not been chemically modified or contacted with a protease, wherein said ECM comprises less than 1% fibronectin or less than 1% laminin by weight; between 74% and 92% Type I collagen by weight; and 4% to 6% Type III collagen or 2% to 15% type IV collagen by weight.

Placental ECM, e.g., ECM comprising placental telopeptide collagen, used in accordance with the methods described herein, may be prepared using methods known in the art, or may be prepared as follows. First, placental tissue (either whole placenta or part thereof) is obtained by standard methods, e.g., collection as soon as practical after Caesarian section or normal birth, e.g., aseptically. The placental tissue can be from any part of the placenta including the amnion, whether soluble or insoluble or both, the chorion, the umbilical cord or from the entire placenta. In certain embodiments, the collagen composition is prepared from whole human placenta without the umbilical cord. The placenta may be stored at room temperature, or at a temperature of about 2° C to 8° C, until further treatment. The placenta is preferably exsanguinated, i.e., completely drained of the placental and cord blood remaining after birth. The expectant mother, in certain embodiments, is screened prior to the time of birth, for, *e.g.,* HIV, HBV, HCV, HTLV, syphilis, CMV, and other viral pathogens known to contaminate placental tissue.

The placental tissue may be decellularized prior to production of the ECM. The placental tissue can be decellularized according to any technique known to those of skill in the art such as those described in detail in U.S. Patent Application Publication Nos. 20040048796 and 20030187515.

The placental tissue may be subjected to an osmotic shock. The osmotic shock can be in addition to any clarification step or it can be the sole clarification step according to the judgment of one of skill in the art. The osmotic shock can be carried out in any osmotic shock conditions known to those of skill in the art. Such conditions include incubating the tissue in solutions of high osmotic potential, or of low osmotic potential or of alternating high and low osmotic potential. The high osmotic potential solution can be any high osmotic potential solution known to those of skill in the art such as a solution comprising one or more of NaCl (e.g., 0.2-1.0 M or 0.2-2.0 M), KCl (e.g., 0.2-1.0 or 0.2 to 2.0 M), ammonium sulfate, a monosaccharide, a disaccharide (e.g., 20% sucrose), a hydrophilic polymer (e.g., polyethylene glycol), glycerol, etc. In certain embodiments, the high osmotic potential solution is a sodium chloride solution, e.g., at least 0.25 M, 0.5M, 0.75M, 11.0M, 1.25M, 1.5M, 1.75M, 2M, or 2.5M NaCl. In some embodiments, the sodium chloride solution is about 0.25-5M, about 0.5-4M, about 0.75-3M, or about 1.0-2.0M NaCl. The low osmotic potential solution can be any low osmotic potential solution known to those of skill in the art, such as water, for example water deionized according to any method known to those of skill. In some embodiments, the osmotic shock solution comprises water with an osmotic shock potential less than that of 50 mM NaCl. In certain embodiments, the osmotic shock is in a sodium chloride solution followed by a water solution. In certain embodiments, one or two NaCl solution treatments are followed by a water wash.

The composition resulting from the osmotic shock may then, in certain embodiments, be incubated with a detergent. The detergent can be any detergent known to those of skill in the art to be capable of disrupting cellular or subcellular membranes, e.g., an ionic detergent, a nonionic detergent, deoxycholate, sodium dodecylsulfate, Triton X 100, TWEEN, or the like. Detergent treatment can be carried out at about 0° C to about 30° C, about 5° C to about 25° C, about 5° C to about 20 ° C, about 5° C to about 15° C, about 0° C, about 5° C, about 10° C, about 15° C, about 20° C, about 25° C., or about 30° C. Detergent treatment can be carried out for, *e.g.,* about 1-24 hours, about 2-20 hours, about 5-15 hours, about 8-12 hours, or about 2-5 hours.

The composition resulting from the detergent treatment may then, in certain embodiments, be incubated under basic conditions. Particular bases for the basic treatment include biocompatible bases, volatile bases, or any organic or inorganic bases at a concentration of, for example, 0.2-1.0M. In certain embodiments, the base is selected from the group consisting of NH₄OH, KOH and NaOH, *e.g.,* 0.1M NaOH, 0.25M NaOH, 0.5M NaOH, or 1M NaOH. The base treatment can be carried out at, *e.g.,* 0° C to 30° C., 5° C to 25° C., 5° C to 20° C., 5° C to 15° C, about 0° C., about 5° C., about 10° C., about 15° C., about 20° C., about 25° C., or about 30° C, for, e.g., about 1-24 hours, about 2-20 hours, about 5-15 hours, about 8-12 hours, or about 2-5 hours.

The ECM can be produced without treatment by a base; omission of a base treatment step typically results in an ECM composition comprising relatively higher amounts of elastin, fibronectin and/or laminin than the ECM composition produced with inclusion of the basic treatment.

Typically, the process described above for human placental tissue results in production of placental ECM comprising base-treated and/or detergent treated Type I telopeptide placental collagen that has not been chemically modified or contacted with a protease, wherein said ECM comprises less than 5% fibronectin or less than 5% laminin by weight; between 25% and 92% Type I collagen by weight; between 2% and 50% Type III collagen; between 2% and 50% type IV collagen by weight; and/or less than 40% elastin by weight. In a more specific embodiment, the process results in production of base-treated, detergent treated Type I telopeptide placental collagen, wherein said collagen has not been chemically modified or contacted with a protease, and wherein said composition comprises less than 1% fibronectin by weight; less than 1% laminin by weight; between 74% and 92% Type I collagen by weight; between 4% and 6% Type III collagen by weight; between 2% and 15% type IV collagen by weight; and/or less than 12% elastin by weight.

In certain embodiments, compositions provided herein that comprise flowable ECM may additionally comprise other components. In certain embodiments, the compositions provided herein that comprise flowable ECM additionally comprise one or more cell types, e.g., one or more of the cell types detailed in Section 4.1.1, above. Alternatively, said cells may be deposited as part of a separate composition in accordance with the methods described herein concurrently with, before, or after the deposition of said ECM.

In certain embodiments, the compositions provided herein that comprise flowable ECM additionally comprise a hydrogel (e.g., a thermosensitive hydrogel and/or a photosensitive hydrogel). Alternatively, a hydrogel may be deposited as part of a separate composition in accordance with the methods described herein concurrently with, before, or after the deposition of said ECM.

In certain embodiments, the compositions provided herein that comprise flowable ECM additionally comprise one or more cell types, e.g., one or more of the cell types detailed in Section 4.1.1, above, and a hydrogel. In a specific embodiment, the compositions provided herein that comprise flowable ECM and a hydrogel (e.g., a thermosensitive hydrogel and/or a photosensitive hydrogel) are formulated such that the ratio of ECM:hydrogel ranges from about 10:1 to about 1:10 by weight.

Exemplary hydrogels may comprise include organic polymers (natural or synthetic) that may be cross-linked via covalent, ionic, or hydrogen bonds to create a three-dimensional open-lattice structure that entraps water molecules to form a gel. Suitable hydrogels for such compositions include self-assembling peptides, such as RAD16. Hydrogel-forming materials include polysaccharides such as alginate and salts thereof, peptides, polyphosphazines, and polyacrylates, which are crosslinked ionically, or block polymers such as polyethylene oxide-polypropylene glycol block copolymers which are crosslinked by temperature or pH, respectively. In some embodiments, the hydrogel or matrix may be biodegradable.

In certain embodiments, the compositions provided herein that comprise flowable ECM additionally comprise a synthetic polymer. In a specific embodiment, the synthetic polymer comprises polyacrylamide, polyvinylidine chloride, poly(o-carboxyphenoxy)-p-xylene) (poly(o-CPX)), poly(lactide-anhydride) (PLAA), n-isopropyl acrylamide, pent erythritol diacrylate, polymethyl acrylate, carboxymethylcellulose, and/or poly(lactic-co-glycolic acid) (PLGA). In another specific embodiment, the synthetic polymer comprises a thermoplastic, e.g., polycaprolactone (PCL), polylactic acid, polybutylene terephthalate, polyethylene terephthalate, polyethylene, polyester, polyvinyl acetate, and/or polyvinyl chloride. Alternatively, one or more synthetic polymers may be deposited as part of a separate composition in accordance with the methods described herein concurrently with, before, or after the deposition of said ECM. In a specific embodiment, the synthetic polymer is PCL.

In certain embodiments, the compositions provided herein that comprise flowable ECM additionally comprise tenascin C, a human protein known to interact with fibronectin, or a fragment thereof. Alternatively, tenascin C may be deposited as part of a separate composition in accordance with the methods described herein concurrently with, before, or after the deposition of said ECM.

In certain embodiments, the compositions provided herein that comprise flowable ECM additionally comprise titanium-aluminum-vanadium (Ti₆Al₄V). Alternatively, Ti₆Al₄V may be deposited as part of a separate composition in accordance with the methods described herein concurrently with, before, or after the deposition of said ECM.

In certain embodiments, the ECM in a composition provided herein and/or an additional component of the composition, such as a synthetic polymer, may be derivatized. Methods for derivatization of ECM and synthetic polymers are known in the art, and include, without limitation, derivatization using cell attachment peptides (e.g., a peptide comprising one or more RGD motifs), derivatization using cell attachment proteins, derivatization using cytokines (e.g., vascular endothelial growth factor (VEGF), or a bone morphogenetic protein (BMP)), and derivatization using glycosaminoglycans.

### 4.1.3.1 Methods of Generating Flowable ECM

The ECM used in accordance with the methods described herein can be made flowable using methods known in the art and described herein.

In one embodiment, the ECM used in accordance with the methods described herein is made flowable by contacting the ECM with an acid or base, e.g., an acidic or basic solution comprising an amount of said acid or base that is sufficient to solubilize said ECM. Once the ECM has been made flowable, if desired, the ECM containing composition can be made neutral, or brought to a desired pH, using methods known in the art.

In another embodiment, the ECM used in accordance with the methods described herein is made flowable by contacting the ECM with an enzyme or combination of enzymes, e.g., a protease, such as trypsin, chymotrypsin, pepsin, papain, and/or elastase. Once the ECM has been made flowable, if desired, the enzymes can be inactivated using methods known in the art.

In another embodiment, the ECM used in accordance with the methods described herein is made flowable using physical approaches. In a specific embodiment, the ECM used in accordance with the methods described herein is made flowable by milling the ECM, i.e., grinding the ECM so as to overcome of the interior bonding forces. In another specific embodiment, the ECM used in accordance with the methods described herein is made flowable by shearing the ECM, e.g., with a blender or other source. In another specific embodiment, the ECM used in accordance with the methods described herein is made flowable by cutting the ECM. In certain embodiments, when ECM is made more flowable by use of physical approaches, the ECM may be manipulated in a frozen state (e.g., the ECM is freeze-dried or frozen in liquid nitrogen).

### 4.1.3.2 Methods of Cross-linking ECM

The ECM used in accordance with the methods described herein can be cross-linked using methods known in the art and described herein.

In certain embodiments, the ECM is cross-linked before it is applied to a surface in or on a subject, i.e., the ECM may be cross-linked before printing. In accordance with such embodiments, a cross-linker may be included in a composition that comprises the ECM and, if necessary, the composition comprising the ECM and cross-linker may be treated under conditions that give rise to the cross-linking of the ECM before the printing of the ECM.

In other embodiments, the ECM is cross-linked after it is applied to a surface in or on a subject, i.e., the ECM may be cross-linked after printing. In one embodiment, the ECM is cross-linked after it is applied to a surface in or on a subject by first printing the ECM onto said surface, followed by printing of a cross-linker to said surface (i.e., the ECM and the cross-linker are printed as separate compositions). In accordance with this embodiment, if necessary, the ECM can subsequently be cross-linked by treating the ECM and cross-linker under conditions that give rise to the cross-linking of the ECM.

In another embodiment, the ECM is cross-linked after it is applied to a surface in or on a subject by printing a composition comprising both the ECM and a cross-linker onto a surface and, after said printing, treating the ECM and cross-linker under conditions that give rise to the cross-linking of the ECM.

In a specific embodiment, the ECM is cross-linked by chemical cross-linking of hyaluronic acid, an ECM component. In another specific embodiment, the ECM is cross-linked by chemical cross-linking of ECM proteins. Exemplary means of chemical cross-linking hyaluronic acid and ECM components include those described in, e.g., Burdick and Prestwich, 2011, Adv. Mater. 23:H41-H56.

In another specific embodiment, the ECM is cross-linked by photopolymerization of hyaluronic acid using, e.g., methacrylic anhydride and/or Glycidyl methacrylate (see, e.g., ., Burdick and Prestwich, 2011, Adv. Mater. 23:H41-H56).

In another specific embodiment, the ECM is cross-linked by the use of enzymes. Enzymes suitable for cross-linking of ECM include, without limitation, lysyl oxidase (see, e.g., Levental et al., 2009, Cell 139:891-906) and tissue type transglutaminases (see, e.g., Griffin et al., 2002, J. Biochem. 368:377-96).

Those of skill in the art will understand the need to select biocompatible chemical cross-linkers, i.e., cross-linkers that have been deemed safe for use in subjects (e.g., human subjects).

### 4.1.4 SURFACES

Any suitable surface in or on a human or animal subject can be used as the surface upon which the cells, flowable ECM, and/or any additional components can be deposited (e.g., printed) on in accordance with methods described herein.

In one embodiment, the surface in or on a subject upon which the cells, flowable ECM, and/or any additional components are deposited comprises an artificial surface that has been transplanted into said subject, i.e., a surface that has been man-made. In a specific embodiment, said artificial surface is a prosthetic. Such artificial surfaces may be selected based on their suitability for administration to and/or transplantation in a subject, e.g., a human subject. For example, an artificial surface known not to be immunogenic (i.e., a surface that does not elicit a host immune response) may be selected for use. In certain embodiments, an artificial surface may be treated so as to render it suitable for administration to and/or transplantation in a subject, e.g., a human subject.

In one embodiment, the surface in or on a subject upon which the cells, flowable ECM, and/or any additional components are deposited comprises a plastic surface. Exemplary types of plastic surfaces onto which said cells, ECM, and/or additional components can be deposited include, without limitation, polyester, polyethylene terephtalate, polyethylene, polyvinyl chloride, polyvinylidene chloride, polypropylene, polystyrene, polyamides, polycarbonate, and polyurethanes.

In one embodiment, the surface in or on a subject upon which the cells, flowable ECM, and/or any additional components are deposited comprises a metal surface. Exemplary types of plastic surfaces onto which said cells, ECM, and/or additional components can be deposited include, without limitation, aluminum, chromium, cobalt, copper, gold, iron, lead, magnesium, manganese, mercury, nickel, platinum, silver, tin, titanium, tungsten, and zinc.

In certain embodiments, the artificial surfaces in or on a subject upon which the cells, flowable ECM, and/or any additional components are deposited are engineered so that they form a particular shape. For example, an artificial surface may be engineered so that is the shape of a bone (e.g., an otic bone), and the appropriate cells (e.g., osteocytes, osteoblasts, osteoclasts and other bone-related cells), flowable ECM, and/or any additional components may be deposited on and/or in said surface.

In another embodiment, said surface in or on a subject comprises a tissue or an organ of a subject (e.g., a human subject). In certain embodiments, the surface in or on a subject of said tissue or organ from a subject may be decellularized, e.g., treated so as to remove cells from all or part of the surface of the tissue or organ.

In another embodiment, said surface in or on a subject is prepared for a method described herein by depositing a composition comprising a biomolecule on said surface prior to said printing, wherein said biomolecule is or comprises a type of collagen, a type of fibronectin, a type of laminin, or a tissue adhesive.

In another embodiment, said surface in or on a subject is prepared for a method described herein by covering all or a portion of said surface with a decellularized tissue, e.g., decellularized amniotic membrane, decellularized diaphragm, decellularized skin, or decellularized fascia.

In accordance with the methods described herein, cells, flowable ECM, and/or any additional components may be deposited on (e.g., printed on) any suitable tissue or organ of a subject. In a specific embodiment, the tissue of the subject that provides the printing surface is connective tissue (including bone), muscle tissue (including visceral (smooth) muscle tissue, skeletal muscle tissue, and cardiac muscle tissue), neural tissue (including central nervous system tissue (e.g., brain tissue or spinal cord tissue) or peripheral nervous system tissue (e.g., cranial nerves and spinal nerves)), or epithelial tissue (including endothelium). In another specific embodiment, the organ of the subject that provides the printing surface is from any of the known mammalian organ systems, including the digestive system, circulatory system, endocrine system, excretory system, immune system, integumentary system, muscular system, nervous system, reproductive system, respiratory system, and/or skeletal system. In another specific embodiment, the organ of a subject that provides the printing surface is all or part of a lung, liver, heart, brain, kidney, skin, bone, stomach, pancreas, bladder, gall bladder, small intestine, large intestine, prostate, testes, ovaries, spinal cord, pharynx, larynx, trachea, bronchi, diaphragm, ureter, urethra, esophagus, colon, thymus, and spleen. In another specific embodiment, a pancreas of a subject that provides the printing surface for the methods described herein.

In a specific embodiment, the cells, flowable ECM, and/or any additional components are deposited on (e.g., printed on) a surface in or on a subject that comprises or consists of bone. Exemplary bones that can be printed on include long bones, short bones, flat bones, irregular bones, and seismoid bones. Specific bones that can be printed on include, without limitation, cranial bones, facial bones, otic bones, bones of the phalanges, arm bones, leg bones, ribs, bones of the hands and fingers, bones of the feet and toes, ankle bones, wrist bones, chest bones (e.g., the sternum), and the like.

In a specific embodiment, the cells, flowable ECM, and/or any additional components are deposited on (e.g., printed on) a surface in or on a subject, wherein the surface is on the exterior of said subject. In a specific embodiment, the cells, flowable ECM, and/or any additional components are deposited on (e.g., printed on) a surface in or on a subject, wherein the surface is within the interior of said individual. In a specific embodiment, the cells, flowable ECM, and/or any additional components are deposited on (e.g., printed on) a surface in or on a subject, wherein the surface is within a body cavity or organ of said individual.

In certain embodiments, the surfaces in or on a subject described herein that serve as scaffolds for the deposition (e.g., deposition by bioprinting or by other means) of cells, flowable ECM, and/or any additional components are surfaces that have not been bioprinted. In certain embodiments, the surfaces in or on a subject described herein that serve as scaffolds for the deposition (e.g., deposition by bioprinting or by other means) of cells, flowable ECM, and/or any additional components are surfaces that have been bioprinted, e.g., bioprinted in accordance with the methods described herein. In a specific embodiment, the bioprinted surface comprises a synthetic material. In a specific embodiment, the synthetic material is PCL.

### 4.2 COMPOSITIONS

Provided herein are compositions that can be used in accordance with the methods described herein. In one embodiment, provided herein are compositions comprising cells (e.g., the cells described in Section 4.1.1, above) that are suitable for use in accordance with the methods described herein. In another embodiment, provided herein are compositions comprising flowable ECM (e.g., the flowable ECM described in Section 4.1.3, above) that is suitable for use in accordance with the methods described herein. In another embodiment, provided are compositions comprising one or more cross-linkers (e.g., the cross-linkers described in Section 4.1.3.2, above) suitable for use in accordance with the methods described herein..

In one embodiment, provided herein is a composition comprising cells (e.g., the cells described in Section 4.1.1, above) and flowable ECM (e.g., the flowable ECM described in Section 4.1.3, above). In a specific embodiment, the cells comprise stem cells, e.g., bone marrow-derived mesenchymal stem cells (BM-MSCs), tissue plastic-adherent placental stem cells (PDACs), and/or amnion derived adherent cells (AMDACs). In another specific embodiment, the flowable ECM is derived from placenta (e.g., human placenta).

In another embodiment, provided herein is a composition comprising flowable ECM (e.g., the flowable ECM described in Section 4.1.3, above) and one or more cross-linkers (e.g., the cross-linkers described in Section 4.1.3.2, above).

In another embodiment, provided herein is a composition comprising cells (e.g., the cells described in Section 4.1.1, above) and one or more cross-linkers (e.g., the cross-linkers described in Section 4.1.3.2, above).

In another embodiment, provided herein is a composition comprising cells (e.g., the cells described in Section 4.1.1, above), flowable ECM (e.g., the flowable ECM described in Section 4.1.3, above), and one or more cross-linkers (e.g., the cross-linkers described in Section 4.1.3.2, above).

In a specific embodiment, a composition provided herein comprises stem cells and flowable ECM, wherein said stem cells are PDACs and wherein said flowable ECM is derived from placenta. In another specific embodiment, a composition provided herein comprises stem cells and a cross-linker, wherein said stem cells are PDACs. In another specific embodiment, a composition provided herein comprises stem cells, flowable ECM, and a cross-linker, wherein said stem cells are PDACs and wherein said flowable ECM is derived from placenta.

In another specific embodiment, a composition provided herein comprises stem cells and flowable ECM, wherein said stem cells are AMDACs and wherein said flowable ECM is derived from placenta. In another specific embodiment, a composition provided herein comprises stem cells and a cross-linker, wherein said stem cells are AMDACs. In another specific embodiment, a composition provided herein comprises stem cells, flowable ECM, and a cross-linker, wherein said stem cells are AMDACs and wherein said flowable ECM is derived from placenta.

In another specific embodiment, a composition provided herein comprises stem cells and flowable ECM, wherein said stem cells are BM-MSCs and wherein said flowable ECM is derived from placenta. In another specific embodiment, a composition provided herein comprises stem cells and a cross-linker, wherein said stem cells are BM-MSCs. In another specific embodiment, a composition provided herein comprises stem cells, flowable ECM, and a cross-linker, wherein said stem cells are BM-MSCs and wherein said flowable ECM is derived from placenta.

The compositions provided herein, in addition to comprising cells (e.g., the cells described in Section 4.1.1, above) and/or flowable ECM (e.g., the flowable ECM described in Section 4.1.3, above) and/or one or more cross-linkers (e.g., the cross-linkers described in Section 4.1.3.2, above) may additionally comprise other components. In certain embodiments, the compositions provided herein additionally comprise a hydrogel (e.g., a thermosensitive hydrogel and/or a photosensitive hydrogel. Alternatively, a hydrogel may be formulated in a composition separate from the cell and ECM comprising compositions provided herein. In certain embodiments, the compositions provided herein additionally comprise a synthetic polymer, such as polyacrylamide, polyvinylidine chloride, poly(o-carboxyphenoxy)-p-xylene) (poly(o-CPX)), poly(lactide-anhydride) (PLAA), n-isopropyl acrylamide, pent erythritol diacrylate, polymethyl acrylate, carboxymethylcellulose, poly(lactic-co-glycolic acid) (PLGA), and/or a thermoplastic (e.g., polycaprolactone, polylactic acid, polybutylene terephthalate, polyethylene terephthalate, polyethylene, polyester, polyvinyl acetate, and/or polyvinyl chloride). Alternatively, a synthetic polymer may be formulated in a composition separate from the cell and ECM comprising compositions provided herein. In certain embodiments, the compositions provided herein additionally comprise tenascin C or a fragment thereof. Alternatively, tenascin C or a fragment thereof may be formulated in a composition separate from the cell and ECM comprising compositions provided herein. In certain embodiments, the compositions provided herein that additionally comprise titanium-aluminum-vanadium (Ti₆Al₄V). Alternatively, Ti₆Al₄V may be formulated in a composition separate from the cell and ECM comprising compositions provided herein.

In certain embodiments, the compositions provided herein additionally comprise one or more additional components that promote the survival, differentiation, proliferation, etc. of the cell(s) used in the compositions. Such components may include, without limitation, nutrients, salts, sugars, survival factors, and growth factors. Exemplary growth factors that may be used in accordance with the methods described herein include, without limitation, insulin-like growth factor (e.g., IGF-1), transforming growth factor-beta (TGF-beta), bone-morphogenetic protein, fibroblast growth factor, platelet derived growth factor (PDGF), vascular endothelial growth factor (VEGF), connective tissue growth factor (CTGF), basic fibroblast growth factor (bFGF), epidermal growth factor, fibroblast growth factor (FGF) (numbers 1, 2 and 3), osteopontin, bone morphogenetic protein-2, growth hormones such as somatotropin, cellular attractants and attachment agents, etc., and mixtures thereof. Alternatively, one or more additional components that promote the survival, differentiation, proliferation, etc. of the cell(s) may be formulated in a composition separate from the cell and ECM comprising compositions provided herein.

### 4.3 USES

The methods described herein can be used for any suitable purpose. In a specific embodiment, the methods described herein are used for therapeutic purposes, e.g., cells, ECM, and/or other additional components are printed in or on a subject so as to result in a therapeutic effect in said subject.

### 4.3.1 Therapeutic Uses

In certain embodiments, the methods described herein are used to treat a subject in need of a transplant (e.g., a tissue or organ transplant). In certain embodiments, the methods described herein are used to treat a subject in need of a graft (e.g., a skin graft). Methods of transplantation, including grafting (e.g., skin grafting) and surgical transplantation procedures are well-known to those of skill in the art and can be modified to conform to the methods described herein.

In certain embodiments, the cells and/or ECM used in the methods described herein are derived are from the transplant recipient. In other embodiments, the cells and/or ECM used in the methods described herein are not from the transplant recipient, but are from another subject, e.g., a donor, a cadaver, etc. In certain embodiments, the cells used in the methods described herein are from the transplant recipient, and the ECM used in the methods described herein is not from the transplant recipient, but is from another source. In a specific embodiment, the ECM used in the methods described herein is derived is from a placenta (e.g., a human placenta). In certain embodiments, the ECM used in the methods described herein is derived is from the transplant recipient, and the cells used in the methods described herein are not from the transplant recipient, but are from another source.

In a specific embodiment, the methods described herein are used to generate epithelial tissue (e.g., skin, dermis, or epidermis) on a surface in or on a subject, wherein said subject is in need of such epithelial tissue (e.g., the subject is a burn victim or has or had a form of skin disease). In a specific embodiment, said subject is human. In another specific embodiment, at least one cellular composition used in the method comprises epidermal cells. In another specific embodiment, at least one cellular composition used in the method comprises dermal cells. In another specific embodiment, at least one cellular composition used in the method comprises mesenchymal stem cells. In another specific embodiment, the cellular composition(s) used in the method comprise epidermal cells, dermal cells, and mesenchymal stem cells. In another specific embodiment, the surface of the subject is a portion of the subject's skin.

In another specific embodiment, the methods described herein are not used to generate epithelial tissue (e.g., skin, dermis, or epidermis) on a surface in or on a subject.

In another specific embodiment, the methods described herein are used to generate connective tissue (e.g., bone) on a surface in or on a subject, wherein said subject is in need of such connective tissue (e.g., the subject has or had osteoporosis or bone cancer). In a specific embodiment, said subject is human. In another specific embodiment, the surface of the subject is one or more of the subject's bones.

In another specific embodiment, the methods described herein are used to generate neural tissue (e.g., brain tissue or spinal cord tissue) on a surface in or on a subject, wherein said subject is in need of such neural tissue. In a specific embodiment, said subject has been diagnosed with a neural disease (i.e., a disease of the central or peripheral nervous system). In another specific embodiment, said subject has suffered trauma that has damaged the central or peripheral nervous system of the subject, e.g., the subject has suffered a traumatic brain injury (TBI) or spinal cord injury (SCI). In another specific embodiment, said subject is human.. In another specific embodiment, the surface of the subject is the subject's brain.. In another specific embodiment, the surface of the subject is the subject's spinal cord.

In another specific embodiment, the methods described herein are used to generate liver tissue on a surface in or on a subject, wherein said subject is in need of such liver tissue

(e.g., the subject has or had cirrhosis of the liver, hepatitis, or liver cancer). In a specific embodiment, said subject is human. In another specific embodiment, the surface of the subject is the subject's liver.

In another specific embodiment, the methods described herein are used to generate lung tissue on a surface in or on a subject, wherein said subject is in need of such lung tissue (e.g., the subject has or had lung cancer, emphysema, or COPD). In a specific embodiment, said subject is human. In another specific embodiment, the surface of the subject is the subject's lung.

In another specific embodiment, the methods described herein are used to generate circulatory system tissue (e.g., heart tissue, arteries, or veins) on a surface in or on a subject, wherein said subject is in need of such circulatory system tissue (e.g., the subject has or had heart disease, coronary artery disease, or issues with the valves of the heart). In a specific embodiment, said subject is human. In another specific embodiment, the surface of the subject is the subject's heart. In another specific embodiment, the surface of the subject is the one or more of the subject's arteries or veins.

In another specific embodiment, the methods described herein are used to generate kidney tissue on a surface in or on a subject, wherein said subject is in need of such kidney tissue (e.g., the subject has or had a form of kidney disease). In a specific embodiment, said subject is human. In another specific embodiment, the cellular composition used in the method comprises at least one type of parenchymal cell and one type of stromal cell. In another specific embodiment, said one type of parenchymal cell and said one type of stromal cells are present in a population of kidney cells disaggregated from autologous or allogeneic kidney tissue. In another specific embodiment, the surface of the subject is one or both of the subject's kidneys.

In another specific embodiment, the methods described herein are used to generate pancreatic tissue on a surface in or on a subject, wherein said subject is in need of such pancreatic tissue (e.g., the subject has or had pancreatic cancer). In a specific embodiment, said subject is human. In another specific embodiment, the surface of the subject is the subject's pancreas.

In another specific embodiment, the methods described herein are used to generate prostate tissue on a surface in or on a subject, wherein said subject is in need of such prostate tissue (e.g., the subject has or had prostate cancer). In a specific embodiment, said subject is human. In another specific embodiment, the surface of the subject is the subject's prostate.

In another specific embodiment, the methods described herein are used to generate stomach tissue on a surface in or on a subject, wherein said subject is in need of such stomach tissue (e.g., the subject has or had stomach cancer). In a specific embodiment, said subject is human. In another specific embodiment, the surface of the subject is the subject's stomach.

In another specific embodiment, the methods described herein are used to generate colon tissue on a surface in or on a subject, wherein said subject is in need of such colon tissue (e.g., the subject has or had colon cancer). In a specific embodiment, said subject is human. In another specific embodiment, the surface of the subject is the subject's colon.

In another specific embodiment, the methods described herein are used to generate intestinal tissue (e.g., one or more tissues associated with the small or large intestine) on a surface in or on a subject, wherein said subject is in need of such intestinal tissue (e.g., the subject has or had a disease of the intestines). In a specific embodiment, said subject is human. In another specific embodiment, the surface of the subject is a portion of the subject's small intestine. In another specific embodiment, the surface of the subject is a portion of the subject's large intestine.

In another specific embodiment, the methods described herein are used to generate esophageal tissue on a surface in or on a subject, wherein said subject is in need of such esophageal tissue. In a specific embodiment, said subject is human. In another specific embodiment, the surface of the subject is the subject's esophagus.

In another specific embodiment, the methods described herein are used to generate thyroid tissue on a surface in or on a subject, wherein said subject is in need of such thyroid tissue. In a specific embodiment, said subject is human. In another specific embodiment, the surface of the subject is the subject's thyroid.

In another specific embodiment, the methods described herein are used to deposit cells in a subject, wherein said cells are engineered to express one or more factors (e.g., proteins or polypeptides), and wherein said subject is in need of said one or more factors, e.g., the subject is deficient in said one or more factors (e.g., due to genetic mutation or genetic predisposition). In a specific embodiment, the subject is a human, e.g., a human with a genetic disease or disorder, wherein said genetic disease or disorder can be treated with, in whole or in part, said one or more factors. In another specific embodiment, the subject is a human with a disease that can be treated with, in whole or in part, said one or more factors. In accordance with such embodiments, the cells can be deposited on any surface, in or on said subject, so long as the one or more factors are expressed by the cell in amount sufficient to treat the disease or disorder in question. Exemplary tissues and organs in or on a subject upon which said cells can be deposited are described in Section 4.1.2, above. Exemplary factors that can be produced by said cells are described in Section 4.1.1, above.

### 4.3.2 Patient Populations

The methods described herein can be used to benefit various patient populations. In one embodiment, the methods described herein are used in subjects that require an organ transplant. In another embodiment, the methods described herein are used in subjects that require treatment of a disease or disorder.

In a specific embodiment, the methods described herein are used to engineer tissue in a subject that has been diagnosed with cancer, i.e., to replace all or part of one or more of the organs/tissues of said subject that have been affected by the cancer. In a specific embodiment, the methods described herein are used to engineer tissue in a subject that has been diagnosed with a bone or connective tissue sarcoma, brain cancer, breast cancer, ovarian cancer, kidney cancer, pancreatic cancer, esophageal cancer, stomach cancer, esophageal cancer, liver cancer, lung cancer (e.g., small cell lung cancer (SCLC), non-small cell lung cancer (NSCLC), throat cancer, and mesothelioma), colon cancer and/or prostate cancer.

In another specific embodiment, the methods described herein are used to engineer tissue in a subject that has been diagnosed with a respiratory disease, e.g., the subject has been diagnosed with asthma, chronic obstructive pulmonary disorder (COPD), emphysema, pneumonia, tuberculosis, lung cancer and/or cystic fibrosis.

In another specific embodiment, the methods described herein are used to engineer tissue in a subject that has been diagnosed with a liver disease, e.g., the subject has been diagnosed with hepatitis (e.g., Hepatitis A, B, or C), liver cancer, hemochromatosis, or cirrhosis of the liver.

In another specific embodiment the methods described herein are used to engineer tissue in a subject that has been diagnosed with bone cancer (e.g., osteosarcoma), osteonecrosis, metabolic bone disease, Fibrodysplasia ossificans progressive, or osteoporosis.

In another specific embodiment, the methods described herein are used to engineer tissue in a subject that has been diagnosed with a neural disease (i.e., a disease of the central or peripheral nervous system), e.g., the subject has been diagnosed with brain cancer, encephalitis, meningitis, Alzheimer's disease, Parkinson's disease, stroke, or multiple sclerosis.

In another specific embodiment, the methods described herein are used to engineer tissue in a subject that has undergone trauma that has damaged the central or peripheral nervous system of the subject, e.g., the subject has suffered a traumatic brain injury (TBI) or spinal cord injury (SCI).

In another specific embodiment, the methods described herein are used to engineer tissue in a subject that has been diagnosed with a disease of the circulatory system, e.g., the subject has been diagnosed with coronary heart disease, cardiomyopathy (e.g., intrinsic or extrinsic cardiomyopathy), heart attack, stroke, inflammatory heart disease, hypertensive heart disease, or valvular heart disease.

In another specific embodiment, the methods described herein are used to engineer tissue in a subject that has been diagnosed with kidney disease. In one embodiment, the subject possesses one kidney that is malfunctioning. In another embodiment, the subject possesses two kidneys that are malfunctioning.

In another specific embodiment, the methods described herein are used to engineer tissue in a subject that has been diagnosed with a genetic disease or disorder, e.g., the subject has been diagnosed with familial hypercholesterolemia, polycystic kidney disease, or phenylketonuria.

In another specific embodiment, the methods described herein are used to engineer tissue in a subject that has been diagnosed with diabetes.

In another specific embodiment, the methods described herein are used to engineer tissue in a subject that has been diagnosed with a cleft palate.

In another specific embodiment, the methods described herein are used to engineer tissue in a subject that undergoes regular procedures that require piercing of the subject's skin (e.g., the subject is a dialysis patient).

In some embodiments, a subject to which a tissue or organ generated in accordance with the methods described herein is transplanted is an animal. In certain embodiments, the animal is a bird. In certain embodiments, the animal is a canine. In certain embodiments, the animal is a feline. In certain embodiments, the animal is a horse. In certain embodiments, the animal is a cow. In certain embodiments, the animal is a mammal, e.g., a horse, swine, mouse, or primate, preferably a human. In a specific embodiment, a subject to which a tissue or organ generated in accordance with the methods described herein is transplanted is a human.

In certain embodiments, a subject to which a tissue or organ generated in accordance with the methods described herein is transplanted is a human adult. In certain embodiments, a subject to which a tissue or organ generated in accordance with the methods described herein is transplanted is a human infant. In certain embodiments, a subject to which a tissue or organ generated in accordance with the methods described herein is transplanted is a human child.

### 4.4 KITS

Provided herein is a pharmaceutical pack or kit comprising one or more containers filled with one or more of the ingredients of the compositions described herein. Optionally associated with such container(s) can be a notice in the form prescribed by a governmental agency regulating the manufacture, use or sale of pharmaceuticals or biological products, which notice reflects approval by the agency of manufacture, use or sale for human administration.

In a specific embodiment, a kit provided herein comprises a composition comprising the cells described herein and the flowable ECM described herein. Such a kit may optionally comprise a composition comprising one or more additional components. The kits encompassed herein can be used in accordance with the methods described herein.

### 5. EXAMPLES

### 5.1 EXAMPLE 1: BIOPRINTED SCAFFOLDS SUPPORT ATTACHMENT AND GROWTH OF PLACENTAL STEM CELLS (comparative, not according to the invention)

This example demonstrates that synthetic material can be bioprinted to produce scaffolds of controlled fiber diameter and pore size, and that such scaffolds provide a suitable substrate for the application of extracellular matrix (ECM). This example further demonstrates that scaffolds comprising bioprinted synthetic material and ECM (hybrid scaffolds) represent a suitable substrate for the attachment and growth of cells, including placental cells, such as placental stem cells.

### 5.1.1 Methods

To fabricate hybrid scaffolds comprising synthetic material and ECM, polycaprolactone (PCL) (Mn 45,000, Sigma) was first printed into scaffolds (54 x 54 x 0.64 mm) using a bioprinter (EnvisionTEC, Gladbeck, Germany). The printing conditions were as follows: temperature at 90°C, printing pressure 3∼5.5 bar, printing speed 2∼6 mm/s, with suitable size needles. ECM was isolated from human placenta as previously described (see, e.g., Bhatia MB, Wounds 20, 29, 2008). Isolated ECM was applied to both sides of the bioprinted PCL scaffolds and allowed to dry (dehydrate) so as to generate hybrid scaffolds comprising PCL and ECM. The resultant hybrid PCL-ECM scaffolds were punched into 10 mm diameter disks, pre-wet with media overnight, and seeded with placental stem cells prepared in accordance with the methods described herein (see, e.g., Section 4.1.1) at 12,500 cells/cm². The cells were cultured over an 8-day time period. Calcein staining and MTS proliferation assays were performed in accordance with standard protocols at different time points (n=3) to determine cell viability and proliferation.

### 5.1.2 Results

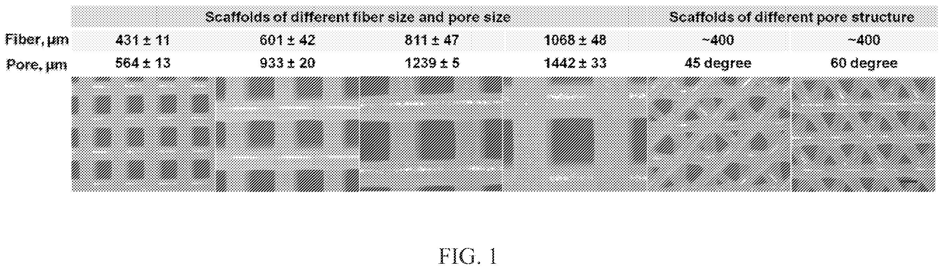

By optimizing printing conditions, PCL scaffolds of different fiber sizes, pore sizes and pore structures were generated (Fig. 1). The printed fibers formed a stable network for the generation of hybrid scaffolds comprising PCL and ECM. Further, the printing of varying fiber sizes and pore structures made it possible to make hybrid scaffolds comprising various properties.

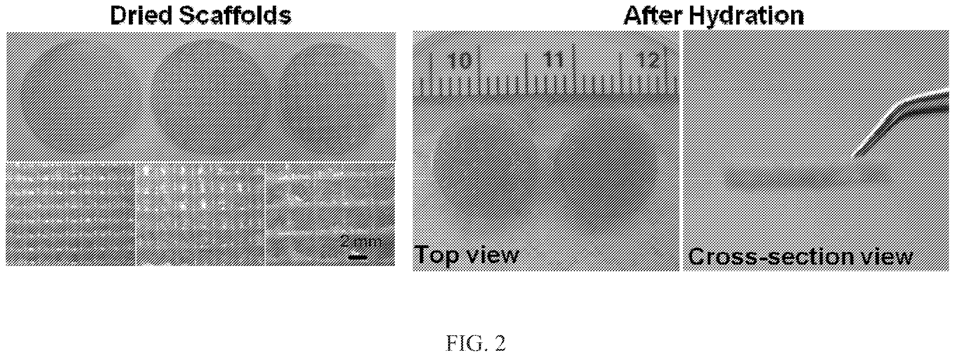

Dehydration of ECM on both sides of the bioprinted PCL scaffolds resulted in the generation of hybrid scaffolds. Good integration was seen between the PCL and ECM; no separation between the PCL and ECM was noticed when the hybrid scaffolds were manipulated by processing or culturing of the scaffolds, which included rehydration (Fig. 2).

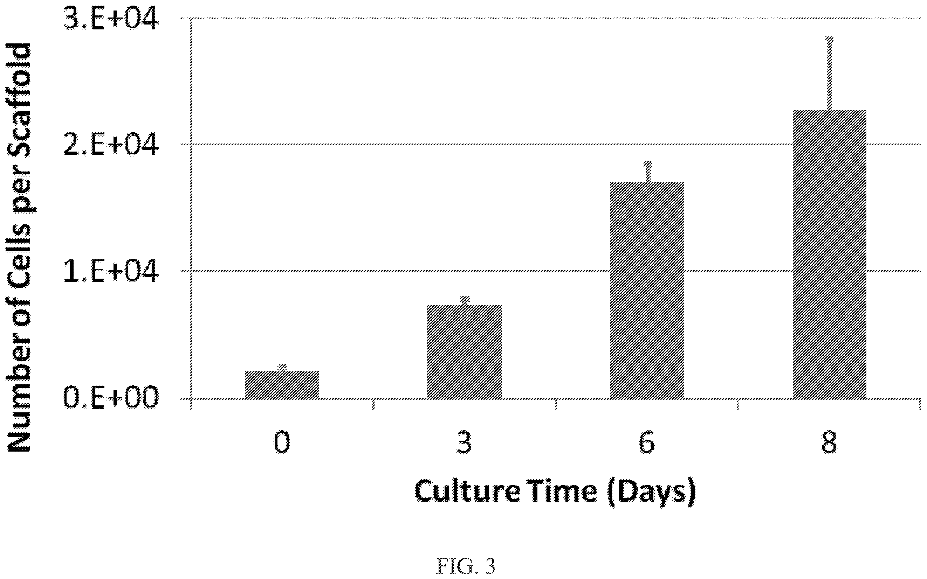

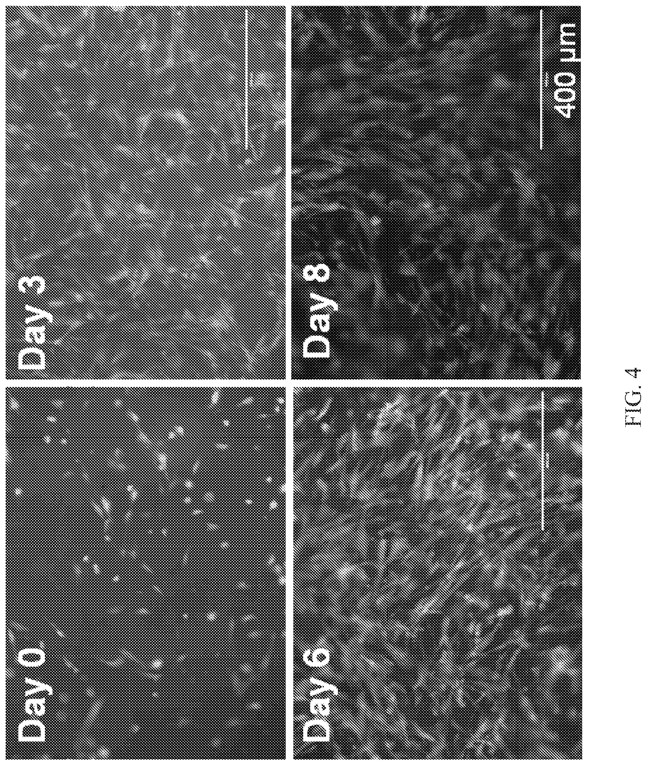

The placental stem cells spread over the surface of the hybrid scaffolds over time, and covered the majority of the surface of the hybrid scaffolds by day 6 of culture. The MTS cell proliferation assay demonstrated that cell number significantly increased over time (Fig. 3). In addition, the placental stem cells seeded on the hybrid scaffolds demonstrated good viability over the 8 day culture period, as indicated by calcein staining (Fig. 4). Together, these data indicate that PCL-ECM hybrid scaffolds support cellular attachment, survival, and growth.

### 5.1.3 Conclusion

This example demonstrates that hybrid scaffolds comprising ECM and synthetic material (PCL) can be generated by methods that comprise bioprinting, and that cells not only attach to such scaffolds, but survive and proliferate when cultured on such scaffolds.

### 5.1 EXAMPLE 2: BIOPRINTED SCAFFOLDS SUPPORT ATTACHMENT AND GROWTH OF PLACENTAL STEM CELLS (comparative, not according to the invention)

This example demonstrates that synthetic material and ECM comprising cells, such as placental cells, e.g., placental stem cells, can be simultaneously bioprinted to produce hybrid scaffolds. As demonstrated by this Example, the bioprinted cells not only survive the bioprinting process, but proliferate over time in culture with the hybrid scaffolds.

### 5.2.1 Methods

ECM was prepared as described in Example 1 and mixed with 0.5% alginate hydrogel containing 1 million/ml placental stem cells. Next, PCL and the cell-containing ECM were bioprinted, in layers, to generate a hybrid scaffold comprising PCL and ECM. In each layer of the scaffold, PCL was first printed, then the ECM/cell component was printed to fill the gaps in between the PCL lines. Two or five of such layers were printed and crosslinked with CaCl₂ solution to generate the hybrid scaffolds. The bioprinted, cell-containing scaffolds (cells/ECM/PCL) were cultured for seven days, and cell proliferation and survival were assessed at various time points via calcein staining and an MTS cell proliferation assay.

### 5.2.2 Results

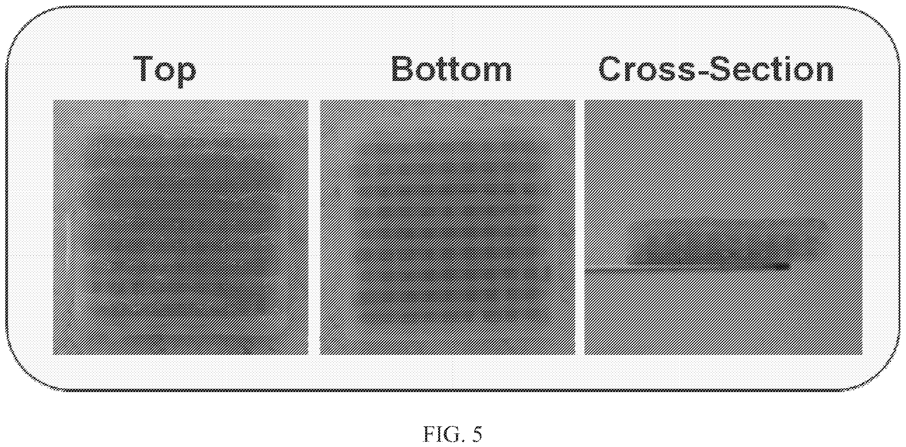

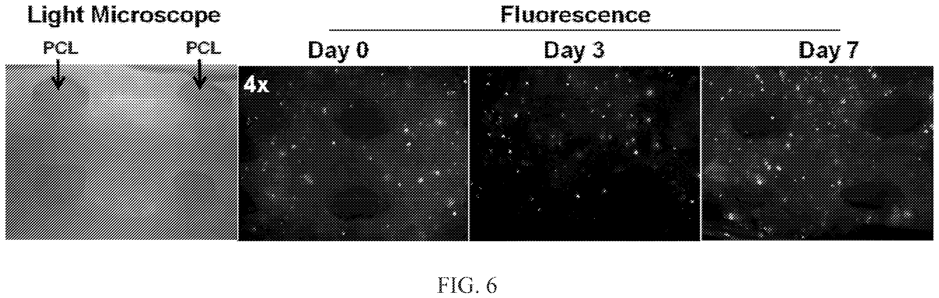

The bioprinted scaffolds maintained an intact structure throughout the duration of cell culture (Fig. 5). PCL provided a good structural support for the ECM hydrogels, which allowed for the generation of three-dimensional constructs. Following bioprinting and throughout culture, the cells were well-distributed throughout the three-dimensional constructs; cells were found throughout the depth of the scaffolds during culture (Fig. 6).

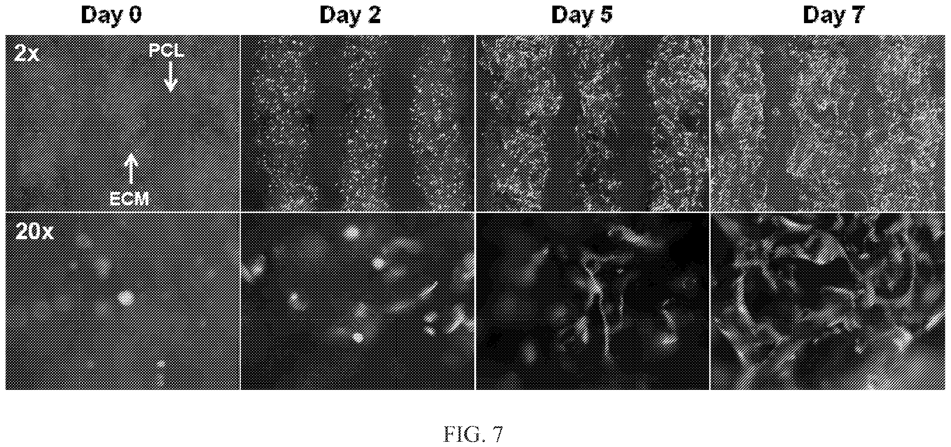

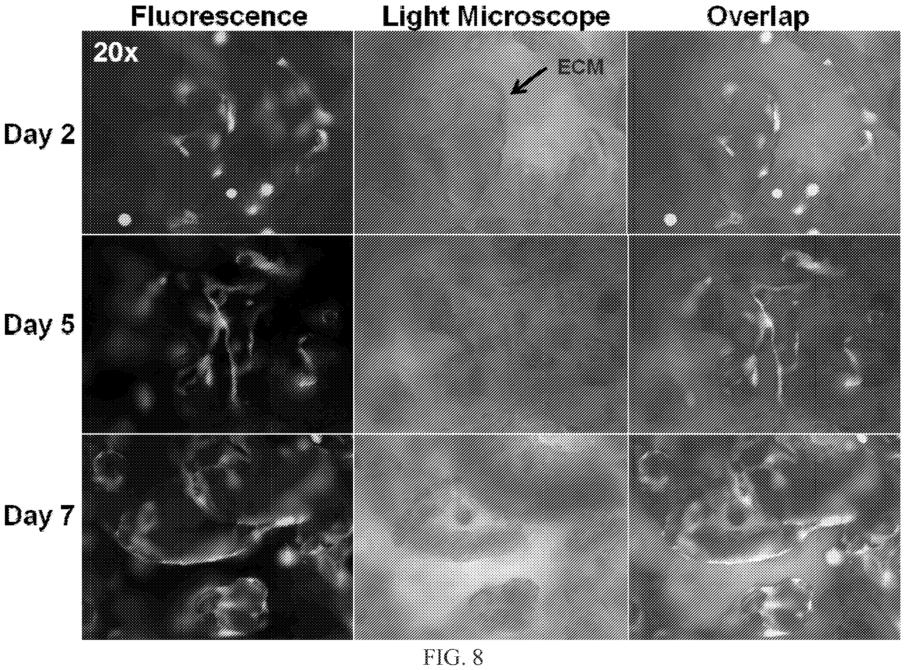

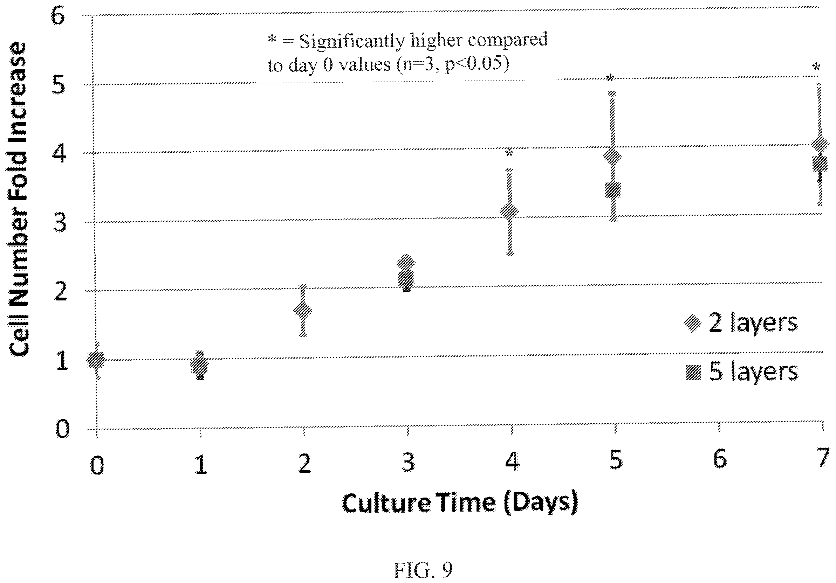

The placental stem cells survived the bioprinting process and continued to proliferate in the three-dimensional bioprinted hybrid scaffolds throughout culture, as evidenced by calcein staining (Fig. 7). As shown in Fig. 8, most of the cells were found to spread throughout the ECM in the hybrid scaffolds, indicating that the ECM enhanced cell attachment and spreading in the ECM hydrogel. This was confirmed by comparing the location of cells in alginate alone with that of the cells in the scaffolds. Additionally, as shown in Figure 9, an MTS cell proliferation assay demonstrated increases in cell number for both the 2-layer and 5-layer scaffolds, indicating that these hybrid scaffolds supported cell growth.

### 5.2.3 Conclusion

This example demonstrates that hybrid scaffolds comprising ECM and synthetic material (PCL) can be generated by methods that comprise simultaneous bioprinting of ECM and PCL. Also demonstrated by this Example is the fact that cells can be bioprinted along with the components of the hybrid scaffold (ECM and PCL), and that the cells survive the bioprinting process. Further, the cells bioprinted along with the components of the hybrid scaffold proliferate when cultured on such scaffolds and intersperse throughout the scaffolds better than when cultured in cellular matrix (alginate) alone.

### 5.3 EXAMPLE 3: FUNCTIONAL BIOPRINTED SCAFFOLDS

This example demonstrates that synthetic material and ECM comprising cells can be bioprinted to produce functional scaffolds.

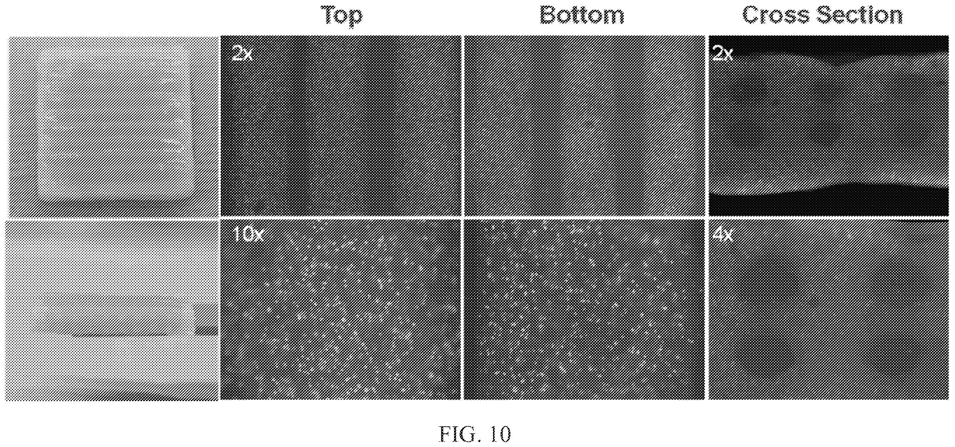

β-TC-6 cells, an insulin producing cell line, were bioprinted with human placenta derived extracellular matrix (ECM) into a bioprinted scaffold. The scaffold was 15 x 15 x 2.5 mm in dimensions, and contained 5 layers. In each layer, polycaprolactone (PCL) was first printed, followed by printing of β-TC-6 cells, mixed at 15 million cells/ml in alginate-ECM hydrogel (1% alginate and 12% ECM) between the PCL lines. The entire scaffold was immersed in 1% calcium chloride solution to crosslink for 20 minutes. The scaffolds then were cultured in DMEM medium containing 15% fetal calf serum in a cell culture incubator in 6 well plates (3 to 5 ml of medium per well). At different time points, the scaffolds were harvested for calcein staining and MTS proliferation assays, to characterize cell viability and cell proliferation, respectively. Figure 10 shows the structure of the bio-printed scaffolds.

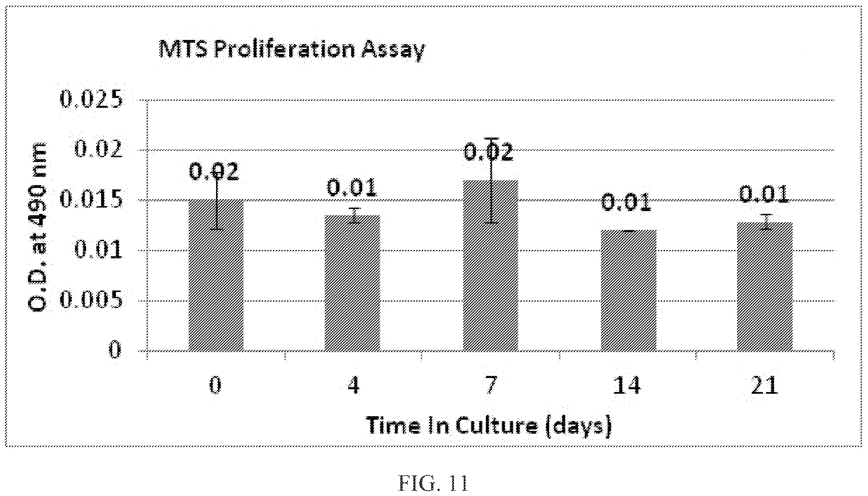

Calcein staining demonstrated that the β-TC-6 cells survived the printing process and remained viable during culture. A cross-sectional view of the scaffolds showed that the cells distributed evenly throughout the scaffolds, and remained alive in each layer (see Figure 10). The MTS assay confirmed that the insulin producing β-TC-6 cells remained viable for up to 3 weeks, with the overall number of viable cells remaining constant (see Figure 11).

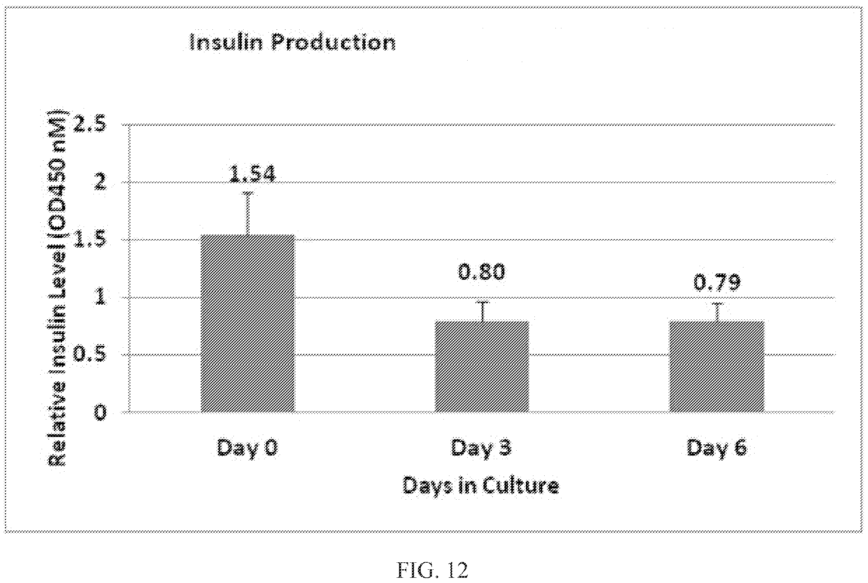

To determine whether the β-TC-6 cells could function in the bioprinted scaffold, insulin production by the cells was measured. To measure insulin production, the bio-printed scaffolds were exposed to fresh growth medium (3 ml/well in a 6-well plate) for 2 hours and aliquots of the supernatant from each scaffold were measured for insulin concentration using a mouse insulin ELISA kit (Millipore). The highest level of insulin produced was detected at day 0 (see Figure 12). The levels of secreted insulin decreased in culture afterwards (day-3 and day-6) but remained stable from day 3 to day 6 in the culture (see Figure 12). Thus, the β-TC-6 cells maintained the ability to produce and secrete insulin after being bioprinted.

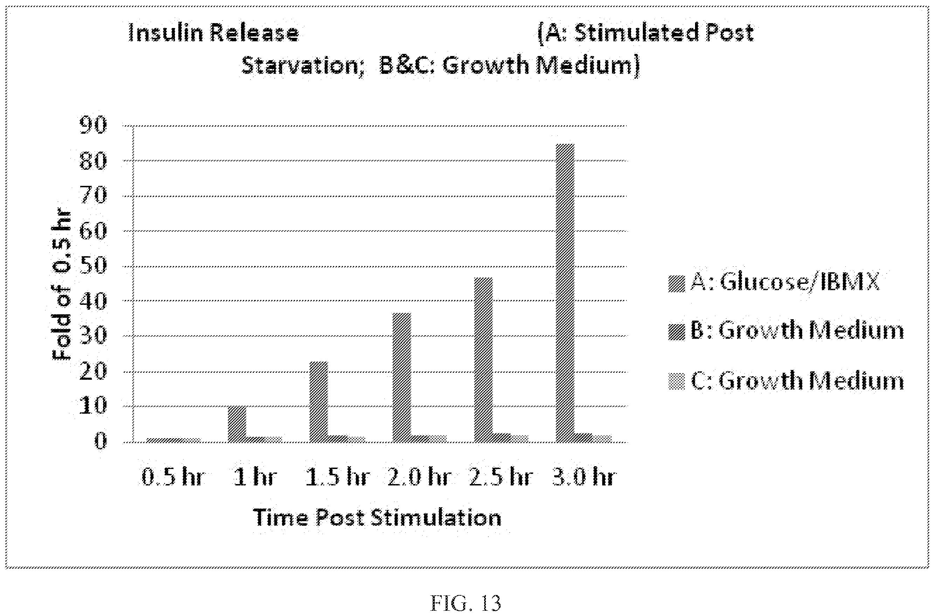

A key function of insulin producing cells in the pancreas is to produce insulin in response to increased glucose levels in the blood. It was thus examined whether the bioprinted scaffolds comprising PCL, ECM, and β-TC-6 cells retained this function by exposing the scaffolds to a glucose surge challenge (see Figure 13). One scaffold ("A" of Figure 13) was exposed to glucose starvation conditions (IMDM medium without glucose, 10% FCS) for two days and then challenged with an insulin producing condition (50 mM glucose/1 mM IBMX). As controls, bioprinted scaffolds were maintained in normal culture medium with steady glucose levels ("B" and "C" of Figure 13). In the controls, the medium was changed at the same time that the challenge with an insulin producing condition was performed for the test scaffold (i.e., A of Figure 13). The supernatant from each culture (A, B, and C) was sampled every half hour and the insulin concentration from each supernatant was measured by ELISA. Figure 13 shows the levels of insulin production from each culture at the different time points and demonstrates that the bioprinted scaffold exposed to glucose starvation conditions followed by challenge with an insulin producing condition (i.e., A of Figure 13) produced greater than 80-fold more insulin after 3 hours after challenge as compared to its level of insulin production at 0.5 hours post-challenge, while the controls (i.e., B and C of Figure 13) produced much less insulin (only approximately 2-fold more insulin after 3 hours following media change as compared to the level of insulin production at 0.5 hour post-media change).

This Example demonstrates that bioprinted scaffolds comprising synthetic material, cells, and ECM can be generated and that the cells of the bioprinted scaffolds remain both viable and functional.

## Claims

1. A cellular composition and placental extracellular matrix (ECM) for use in a method of treatment of a human or animal body, wherein the method comprises forming a three-dimensional tissue comprising depositing the cellular composition and placental ECM onto a surface in or on a human or animal subject, wherein the cellular composition comprises insulin-producing cells, and wherein said cellular composition and said ECM are both printed onto said surface.

2. The cellular composition and placental extracellular matrix (ECM) for use in a method of claim 1, wherein said surface is an artificial surface, a decellularized tissue or a decellularized organ.

3. The cellular composition and placental extracellular matrix (ECM) for use in a method of claim 2, wherein said artificial surface is a prosthetic device or structure.

4. The cellular composition and placental extracellular matrix (ECM) for use in a method of any of claims 1-4, wherein said surface is a surface on the exterior of said subject, or wherein said surface is a surface within the interior of said subject.

5. The cellular composition and placental extracellular matrix (ECM) for use in a method of any of claims 1-5, comprising scanning said surface in or on said subject so as to form a surface map, and using said surface map to guide said depositing, preferably, wherein said scanning comprises the use of a laser, electron beam, magnetic resonance imaging, microwave, x-ray, or computed tomography.

6. The cellular composition and placental extracellular matrix (ECM) for use in a method of any of claims 1-5, further comprising deposition of a hydrogel, a synthetic polymer, tenascin C or a fragment thereof, and/or a titanium-aluminum-vanadium (Ti₆Al₄V) composition.

7. The cellular composition and placental extracellular matrix (ECM) for use in a method of any of claims 1-6 wherein said cellular composition further comprises
(i) bone marrow-derived mesenchymal stem cells (BM-MSCs);
(ii) tissue culture plastic-adherent CD34-, CD10+, CD105+, CD200+ placental stem cells;
(iii) embryonic stem cells, induced pluripotent stem cells, mesenchymal stem cells, bone marrow-derived mesenchymal stem cells, bone marrow derived mesenchymal stromal cells, tissue plastic-adherent placental stem cells (PDACs), umbilical cord stem cells, amniotic fluid stem cells, amnion derived adherent cells (AMDACs), osteogenic placental adherent cells (OPACs), adipose stem cells, limbal stem cells, dental pulp stem cells, myoblasts, endothelial progenitor cells, neuronal stem cells, exfoliated teeth derived stem cells, hair follicle stem cells, dermal stem cells, parthenogenically derived stem cells, reprogrammed stem cells, amnion derived adherent cells, or side population stem cells; and/or
(iv) differentiated cells.

8. The cellular composition and placental extracellular matrix (ECM) for use in a method of claim 7, wherein said differentiated cells comprise endothelial cells, epithelial cells, dermal cells, endodermal cells, mesodermal cells, fibroblasts, osteocytes, chondrocytes, natural killer cells, dendritic cells, hepatic cells, pancreatic cells, or stromal cells;
salivary gland mucous cells, salivary gland serous cells, von Ebner's gland cells, mammary gland cells, lacrimal gland cells, ceruminous gland cells, eccrine sweat gland dark cells, eccrine sweat gland clear cells, apocrine sweat gland cells, gland of Moll cells, sebaceous gland cells, bowman's gland cells, Brunner's gland cells, seminal vesicle cells, prostate gland cells, bulbourethral gland cells, Bartholin's gland cells, gland of Littre cells, uterus endometrium cells, isolated goblet cells, stomach lining mucous cells, gastric gland zymogenic cells, gastric gland oxyntic cells, pancreatic acinar cells, paneth cells, type II pneumocytes, clara cells,
somatotropes, lactotropes, thyrotropes, gonadotropes, corticotropes, intermediate pituitary cells, magnocellular neurosecretory cells, gut cells, respiratory tract cells, thyroid epithelial cells, parafollicular cells, parathyroid gland cells, parathyroid chief cell, oxyphil cell, adrenal gland cells, chromaffin cells, Leydig cells, theca intema cells, corpus luteum cells, granulosa lutein cells, theca lutein cells, juxtaglomerular cell, macula densa cells, peripolar cells, mesangial cell,
blood vessel and lymphatic vascular endothelial fenestrated cells, blood vessel and lymphatic vascular endothelial continuous cells, blood vessel and lymphatic vascular endothelial splenic cells, synovial cells, serosal cell (lining peritoneal, pleural, and pericardial cavities), squamous cells, columnar cells, dark cells, vestibular membrane cell (lining endolymphatic space of ear), stria vascularis basal cells, stria vascularis marginal cell (lining endolymphatic space of ear), cells of Claudius, cells of Boettcher, choroid plexus cells, pia-arachnoid squamous cells, pigmented ciliary epithelium cells, nonpigmented ciliary epithelium cells, corneal endothelial cells, peg cells,
respiratory tract ciliated cells, oviduct ciliated cell, uterine endometrial ciliated cells, rete testis ciliated cells, ductulus efferens ciliated cells, ciliated ependymal cells,
epidermal keratinocytes, epidermal basal cells, keratinocyte of fingernails and toenails, nail bed basal cells, medullary hair shaft cells, cortical hair shaft cells, cuticular hair shaft cells, cuticular hair root sheath cells, hair root sheath cells of Huxley's layer, hair root sheath cells of Henle's layer, external hair root sheath cells, hair matrix cells,
surface epithelial cells of stratified squamous epithelium, basal cell of epithelia, urinary epithelium cells,
auditory inner hair cells of organ of Corti, auditory outer hair cells of organ of Corti, basal cells of olfactory epithelium, cold-sensitive primary sensory neurons, heat-sensitive primary sensory neurons, Merkel cells of epidermis, olfactory receptor neurons, pain-sensitive primary sensory neurons, photoreceptor rod cells, photoreceptor blue-sensitive cone cells, photoreceptor green-sensitive cone cells, photoreceptor red-sensitive cone cells, proprioceptive primary sensory neurons, touch-sensitive primary sensory neurons, type I carotid body cells, type II carotid body cell (blood pH sensor), type I hair cell of vestibular apparatus of ear (acceleration and gravity), type II hair cells of vestibular apparatus of ear, type I taste bud cells
cholinergic neural cells, adrenergic neural cells, peptidergic neural cells,
inner pillar cells of organ of Corti, outer pillar cells of organ of Corti, inner phalangeal cells of organ of Corti, outer phalangeal cells of organ of Corti, border cells of organ of Corti, Hensen cells of organ of Corti, vestibular apparatus supporting cells, taste bud supporting cells, olfactory epithelium supporting cells, Schwann cells, satellite cells, enteric glial cells,
astrocytes, neurons, oligodendrocytes, spindle neurons,
anterior lens epithelial cells, crystallin-containing lens fiber cells, hepatocytes, adipocytes, white fat cells, brown fat cells, liver lipocytes,
kidney glomerulus parietal cells, kidney glomerulus podocytes, kidney proximal tubule brush border cells, loop of Henle thin segment cells, kidney distal tubule cells, kidney collecting duct cells, type I pneumocytes, pancreatic duct cells, nonstriated duct cells, duct cells, intestinal brush border cells, exocrine gland striated duct cells, gall bladder epithelial cells, ductulus efferens nonciliated cells, epididymal principal cells, epididymal basal cells,
ameloblast epithelial cells, planum semilunatum epithelial cells, organ of Corti interdental epithelial cells, loose connective tissue fibroblasts, corneal keratocytes, tendon fibroblasts, bone marrow reticular tissue fibroblasts, nonepithelial fibroblasts, pericytes, nucleus pulposus cells, cementoblast/cementocytes, odontoblasts, odontocytes, hyaline cartilage chondrocytes, fibrocartilage chondrocytes, elastic cartilage chondrocytes, osteoblasts, osteocytes, osteoclasts, osteoprogenitor cells, hyalocytes, stellate cells (ear), hepatic stellate cells (Ito cells), pancreatic stelle cells,
red skeletal muscle cells, white skeletal muscle cells, intermediate skeletal muscle cells, nuclear bag cells of muscle spindle, nuclear chain cells of muscle spindle, satellite cells, ordinary heart muscle cells, nodal heart muscle cells, Purkinje fiber cells, smooth muscle cells, myoepithelial cells of iris, myoepithelial cell of exocrine glands,
reticulocytes, megakaryocytes, monocytes, connective tissue macrophages, epidermal Langerhans cells, dendritic cells, microglial cells, neutrophils, eosinophils, basophils, mast cell, helper T cells, suppressor T cells, cytotoxic T cell, natural Killer T cells, B cells, natural killer cells,
melanocytes, retinal pigmented epithelial cells, Sertoli cells, thymus epithelial cell, and/or interstitial kidney cells.

9. The cellular composition and placental extracellular matrix (ECM) for use in a method of any of claims 1-8, wherein cells in said cellular composition are primary culture cells, preferably from said subject; or cells that have been cultured *in vitro.*

10. The cellular composition and placental extracellular matrix (ECM) for use in a method of any of claims 1-9, wherein said cells have been genetically engineered to produce a protein or polypeptide not naturally produced by the cell, or have been genetically engineered to produce a protein or polypeptide in an amount greater than that naturally produced by the cell, wherein said cellular composition comprises differentiated cells.

11. The cellular composition and placental extracellular matrix (ECM) for use in a method of claim 10, wherein 1 x 10⁶ of said cells produces at least 1.0 to 10 µM of said protein in *in vitro* culture in growth medium over 24 hours.

12. The cellular composition and placental extracellular matrix (ECM) for use in a method of claim 10, wherein said protein is a protein missing or malfunctioning in a subject who has a genetic disorder or disease, preferably wherein:
said genetic disease is familial hypercholesterolemia and said protein is low density lipoprotein receptor (LDLR);
said genetic disease is polycystic kidney disease, and said protein is polycystin-1 (PKD1), PKD-2 or PKD3; or
said genetic disease is phenylketonuria and said protein is phenylalanine hydroxylase.

13. The cellular composition and placental extracellular matrix (ECM) for use in a method of claim 1, wherein said surface is prepared by depositing a composition comprising a biomolecule on said surface prior to said printing, preferably wherein said biomolecule is or comprises a type of collagen, a type of fibronectin, a type of laminin, or a tissue adhesive; or
wherein said surface is prepared by covering all or a portion of said surface with a decellularized tissue, preferably,
wherein said decellularized tissue is decellularized amniotic membrane, decellularized diaphragm, decellularized skin, or decellularized fascia.

14. The cellular composition and placental extracellular matrix (ECM) for use in a method of any of claims 1-13, wherein said surface is a surface of a heart, blood vessel, skin, liver, pancreas, lung, kidney, thyroid, section of intestine, stomach, trachea, esophagus, or duodenum in said subject; or wherein said surface is damaged skin.

15. The cellular composition and placental extracellular matrix (ECM) for use in a method of any of claims 1-14, wherein said depositing is accomplished by inkjet printing.

16. The cellular composition and placental extracellular matrix (ECM) for use in a method of claim 1, wherein said surface is not skin, epidermis, or dermis.

## Patentansprüche

1. Zellzusammensetzung und plazentale extrazelluläre Matrix (ECM) zur Verwendung in einem Behandlungsverfahren eines menschlichen oder tierischen Körpers, wobei das Verfahren das Bilden eines dreidimensionalen Gewebes, umfassend das Ablagern der Zellzusammensetzung und der plazentalen ECM auf einer Oberfläche in oder auf einem menschlichen oder tierischen Individuum, umfasst, wobei die Zellzusammensetzung Insulin produzierende Zellen umfasst und wobei die Zellzusammensetzung und die ECM beide auf die Oberfläche gedruckt werden.

2. Zellzusammensetzung und plazentale extrazelluläre Matrix (ECM) zur Verwendung in einem Verfahren nach Anspruch 1, wobei die Oberfläche eine künstliche Oberfläche, ein dezellularisiertes Gewebe oder ein dezellularisiertes Organ ist.

3. Zellzusammensetzung und plazentale extrazelluläre Matrix (ECM) zur Verwendung in einem Verfahren nach Anspruch 2, wobei die künstliche Oberfläche eine prosthetische Vorrichtung oder Struktur ist.

4. Zellzusammensetzung und plazentale extrazelluläre Matrix (ECM) zur Verwendung in einem Verfahren nach einem der Ansprüche 1 bis 4, wobei die Oberfläche eine Oberfläche auf der Außenseite des Individuums ist oder wobei die Oberfläche eine Oberfläche im Inneren des Individuums ist.

5. Zellzusammensetzung und plazentale extrazelluläre Matrix (ECM) zur Verwendung in einem Verfahren nach einem der Ansprüche 1 bis 5, umfassend das Scannen der Oberfläche in oder auf dem Individuum, um eine Oberflächenkarte zu bilden, und das Verwenden der Oberflächenkarte, um das Ablagern anzuleiten, wobei das Scannen vorzugsweise die Verwendung eines/einer Lasers, Elektronenstrahls, Magnetresonanzbildgebung, Mikrowelle, Röntgen oder Computertomographie umfasst.

6. Zellzusammensetzung und plazentale extrazelluläre Matrix (ECM) zur Verwendung in einem Verfahren nach einem der Ansprüche 1 bis 5, ferner umfassend das Ablagern eines Hydrogels, eines synthetischen Polymers, eines Tenascin C oder eines Fragments davon und/oder einer Titan-Aluminium-Vanadium- (Ti₆Al₄V-) Zusammensetzung.

7. Zellzusammensetzung und plazentale extrazelluläre Matrix (ECM) zur Verwendung in einem Verfahren nach einem der Ansprüche 1 bis 6, wobei die Zellzusammensetzung ferner Folgendes umfasst:
(i) aus dem Knochenmark stammende mesenchymale Stammzellen (BM-MSCs);
(ii) an Kunststoff haftende Gewebekultur-CD34--, -CD10+-, -CD105+-, - CD200+-Plazentastammzellen;
(iii) embryonale Stammzellen, induzierte pluripotente Stammzellen, mesenchymale Stammzellen, aus dem Knochenmark stammende mesenchymale Stammzellen, aus dem Knochenmark stammende mesenchymale Stromazellen, an Kunststoff haftende Gewebekultur-Plazentastammzellen (PDACs), Nabelschnurstammzellen, Fruchtwasserstammzellen, aus dem Amnion stammende haftende Zellen (AMDACs), osteogene haftende Plazentazellen (OPACs), adipöse Stammzellen, Limbusstammzellen, Zahnmarkstammzellen, Myoblasten, endotheliale Vorläuferzellen, neuronale Stammzellen, exfoliierte von Zähnen stammende Stammzellen, Haarfollikelstammzellen, Hautstammzellen, von Parthenogenese stammende Stammzellen, umprogrammierte Stammzellen, von Amnion stammende haftende Zellen oder Seitenpopulationsstammzellen; und oder
(iv) differenzierte Zellen.

8. Zellzusammensetzung und plazentale extrazelluläre Matrix (ECM) zur Verwendung in einem Verfahren nach Anspruch 7, wobei die differenzierten Zellen Endothelzellen, Epithelzellen, Hautzellen, Endodermzellen, Mesodermzellen, Fibroblasten, Osteozyten, Chondrozyten, natürliche Killerzellen, dendritische Zellen, Leberzellen, Bauchspeicheldrüsenzellen oder Stromazellen;
Speicheldrüsenschleimhautzellen, Speicheldrüsenserumzellen, von-Ebner-Drüsenzellen, Brustdrüsenzellen, Tränendrüsenzellen, Zeruminaldrüsenzellen, ekkrine Schweißdrüsen-Dunkelzellen, ekkrine Schweißdrüsen-Klarzellen, apokrine Schweißdrüsenzellen, Moll-Drüsenzellen, Talgdrüsenzellen, Bowman-Drüsenzellen, Brunner-Drüsenzellen, Bläschendrüsenzellen, Prostatazellen, Bulbourethraldrüsenzellen, Bartholin-Drüsenzellen, Littré-Drüsenzellen, Gebärmutter- (Endometrium-) Zellen, isolierte Becherzellen, Magenschleimhautzellen, enzymbildende Magendrüsenzellen, säurebildende Magendrüsenzellen, Bauchspeicheldrüsen-Azinuszellen, Paneth-Zellen, Typ-II-Pneumozyten, Club-Zellen,
somatotrope, laktotrope, thyrotrope, gonadotrope, kortikotrope Zellen, Hypophyse-Zwischenzellen, magnozelluläre neurosekretorische Zellen, Darmzellen, Atemwegszellen, Schilddrüsenepithelzellen, parafollikuläre Zellen, Nebenschilddrüsenzellen, Nebennierenhauptzelle, oxyphile Zelle, Nebennierenzellen, Chromaffinzellen, Leydig-Zellen, *Theca-interna*-Zellen, *Corpus-luteum*-Zellen, *Granulosa-lutein*-Zellen, *Theca-lutein*-Zellen, juxtaglomeruläre Zelle, *Makula-densa*-Zellen, peripolare Zellen, Mesangiumzellen,
Blutgefäß- und lymphatische gefensterte Gefäßendothelzellen, Blutgefäß- und lymphatische kontinuierliche Gefäßendothelzellen, Blutgefäße und lymphatische Gefäßendothelmilzzellen, Synovialzellen, Serosazellen (Auskleidung von Peritoneal-, Brust- und Perikardhöhlen), Plattenepithelzellen, hochprismatische Zellen, Dunkelzellen, Vestibularmembranzellen (Auskleidung endolymphatischen Raums des Ohrs), *Stria-vascularis*-Basalzellen, *Stria-vascularis*-Randzellen (Auskleidung des endolymphatischen Raumes des Ohrs), Claudius-Zellen, Boettcher-Zellen, *Plexus-choroideus*-Zellen, *Pia-Arachnoidea*-Plattenepithelzellen, pigmentierte Ziliarepithelzellen, nicht pigmentierte Ziliarepithelzellen, korneale Endothelzellen, Peg-Zellen,
Flimmerzellen des Respirationstraktes, Flimmerzellen der Eizelle, Flimmerzellen der Gebärmutter (Endometrium), Flimmerzellen des *Rete testis,* Flimmerzellen des *Ductulus efferens,* Flimmer-Ependymzellen,
epidermale Keratinozyten, epidermale Basalzellen, Keratinozyt der Finger- und Fußnägel, Nagelbett-Basalzellen, Markhaarschaftszellen, cortikale Haarschaftszellen, kutikuläre Haarschaftszellen, kultikuläre Haarwurzelscheidenzellen, Haarwurzelscheidenzellen der Huxley-Schicht, Haarwurzelschalenzellen der Henle-Schicht, externe Haarwurzelscheidenzellen, Haarmatrixzellen,
Oberflächenepithelzellen aus geschichtetem Plattenepithel, Basalzellen der Epithelien, Harnepithelzellen,
innere Gehörhaarzellen des Corti-Organs, äußere Gehörhaarzellen des Corti-Organs, Basalzellen des Riechepithels, kälteempfindliche primäre sensorische Neuronen, wärmeempfindliche primäre sensorische Neuronen, Merkelzellen der Epidermis, Geruchsrezeptor-Neuronen, schmerzempfindliche primäre sensorische Neuronen, Fotorezeptor-Stäbchenzellen, blauempfindliche Fotorezeptor-Zapfenzellen, grünempfindliche Fotorezeptor-Zapfenzellen, rotempfindliche Fotorezeptor-Zapfenzellen, propriozeptive primäre sensorische Neuronen, berührungsempfindliche primäre sensorische Neuronen, Typ-I-Halsschlagaderzellen, Typ-II-Halsschlagaderzellen (pH-Sensor Blut), Typ-I-Haarzelle des Gleichgewichtsorgans des Ohrs (Beschleunigung und Schwerkraft), Typ-II-Haarzellen des Gleichgewichtsorgans des Ohrs, Typ-I-Geschmacksknospenzellen,
cholinerge neurale Zellen, adrenerge neurale Zellen, peptiderge neurale Zellen, innere Säulenzellen des Corti-Organs, äußere Säulenzellen des Corti-Organs, innere Phalangenzellen des Corti-Organs, äußere Phalangenzellen des Corti-Organs, Randzellen des Corti-Organs, Hensen-Zellen des Corti-Organs, das Gleichgewichtsorgan unterstützende Zellen, Geschmacksknospen unterstützende Zellen, olfaktorisches Epithel unterstützende Zellen, Schwann-Zellen, Satellitenzellen, Darmgliazellen,
Astrozyten, Neuronen, Oligodendrozyten, Spindelneuronen,
vordere Linsenepithelzellen, kristallinhaltige Linsenfaserzellen,
Hepatozyten, Adipozyten, weiße Fettzellen, braune Fettzellen, Leberlipozyten, Nierenglomerulus-Parietalzellen, Podozyten der Nierenglomeruli, Bürstensaumzelle der proximalen Tubuli der Niere, Dünnes-Segment-Zelle der Henleschen Schleife, Nierenzelle der distalen Tubuli der Niere, Nierensammelrohrzellen, Typ-I-Pneumozyten, Pankreasgangzellen, glatte Kanalzellen, Kanalzellen, Darmbürstenrandzellen, quergestreifte Hormondrüsen-Duktalzellen, Gallenblasenepithelzellen, unbegeißelte Zelle des *Ductulus efferens,* Nebenhodenhauptzellen, Nebenhodenbasalzellen,
Ameloblast-Epithelzellen, *Planum-semilunatum*-Epithelzellen, interdentale Epithelzellen des Corti-Organs, Fibroblasten des weichen Bindegewebes, korneale Keratozyten, Sehnenfibroblasten, retikuläre Gewebefibroblasten aus dem Knochenmark, nichtepitheliale Fibroblasten, Perizyten, *Nucleus-pulposus*-Zellen, Zementoblasten/Zementozyten, Odontoblasten, Odontozyten, Chondrozyten der Hyalinknorpel, Chondrozyten der Faserknorpel, Chondrozyten der elastischen Knorpel, Osteoblasten, Osteozyten, Osteoklasten, Osteovorläuferzellen, Hyalozyten, Sternzellen (Ohr), hepatische Sternzellen (Ito-Zellen), Bauchspeicheldrüsenzellen,
rote Skelettmuskelzellen, weiße Skelettmuskelzellen, Zwischenskelettmuskelzellen, Kernsackzellen der Muskelspindel, Kernkettenzellen der Muskelspindel, Satellitenzellen, gewöhnliche Herzmuskelzellen, Knotenherzmuskelzellen, Purkinje-Faserzellen, glatte Muskelzellen, Myoepithelzellen der Iris, Myoepithelzellen exokriner Drüsen,
Retikulozyten, Megakaryozyten, Monozyten, Bindegewebsmakrophagen, epidermale Langerhans-Zellen, dendritische Zellen, Mikrogliazellen, Neutrophile, Eosinophile, Basophile, Mastzelle, T-Helferzellen, Suppressor-T-Zellen, zytotoxische T-Zellen, natürliche Killer-T-Zellen, B-Zellen, natürliche Killer-Zellen,
Melanozyten, retinale pigmentierte Epithelzellen,
Sertolizellen, Thymusepithelzellen und/oder interstitielle Nierenzellen umfassen.

9. Zellzusammensetzung und plazentale extrazelluläre Matrix (ECM) zur Verwendung in einem Verfahren nach einem der Ansprüche 1 bis 8, wobei in der Zellzusammensetzung Primärkulturzellen, vorzugsweise von dem Individuum, oder Zellen, die *in vitro* kultiviert wurden, vorliegen.

10. Zellzusammensetzung und plazentale extrazelluläre Matrix (ECM) zur Verwendung in einem Verfahren nach einem der Ansprüche 1 bis 9, wobei die Zellen genetisch verändert wurden, um ein Protein oder ein Polypeptid herzustellen, das von der Zelle nicht natürlich hergestellt wird, oder genetisch verändert wurden, um ein Protein oder Polypeptid in einer Menge herzustellen, die größer ist als jene Menge, die von der Zelle natürlich hergestellt wird, wobei die Zellzusammensetzung differenzierte Zellen umfasst.

11. Zellzusammensetzung und plazentale extrazelluläre Matrix (ECM) zur Verwendung in einem Verfahren nach Anspruch 10, wobei 1 x 10⁶ der Zellen zumindest 1,0 bis 10 µM des Proteins in *In-vitro*-Kultur im Wachstumsmedium über 24 h herstellen.

12. Zellzusammensetzung und plazentale extrazelluläre Matrix (ECM) zur Verwendung in einem Verfahren nach Anspruch 10, wobei das Protein ein Protein ist, das in einem Individuum, das eine genetische Störung oder Krankheit aufweist, fehlt oder nicht richtig funktioniert, wobei vorzugsweise:
die genetische Störung eine familiäre Hypercholesterinämie und das Protein ein Lipoproteinrezeptor niedriger Dichte (LDLR) ist;
die genetische Störung eine polyzystische Nierenerkrankung und das Protein ein Polycystin-1 (PKD1), PKD-2 oder PKD3 ist; oder
die genetische Störung Phenylketonurie und das Protein Phenylalaninhydroxylase ist.

13. Zellzusammensetzung und plazentale extrazelluläre Matrix (ECM) zur Verwendung in einem Verfahren nach Anspruch 1, wobei die Oberfläche durch Ablagerung einer Zusammensetzung, umfassend ein Biomolekül, auf der Oberfläche vor dem Drucken hergestellt ist, wobei das Biomolekül vorzugsweise eine Art Kollagen, eine Art Fibronectin, eine Art Laminin oder ein Gewebekleber ist oder eine/n solche/n umfasst, oder
wobei die Oberfläche durch das Abdecken der gesamten oder eines Teils der Oberfläche mit einem dezellularisierten Gewebe hergestellt ist,
wobei das dezellularisierte Gewebe vorzugsweise dezellularisierte amniotische Membran, dezellularisiertes Diaphragma, dezellularisierte Haut oder dezellularisierte Faszie ist.

14. Zellzusammensetzung und plazentale extrazelluläre Matrix (ECM) zur Verwendung in einem Verfahren nach einem der Ansprüche 1 bis 13, wobei die Oberfläche eine Oberfläche eines/einer Herzen, Blutgefäßes, Haut, Leber, Bauchspeicheldrüse, Lunge, Niere, Schilddrüse, Abschnitts des Darms, Magens, Luftröhre, Speiseröhre oder Zwölffingerdarms in einem Individuum ist; oder wobei die Oberfläche verletzte Haut ist.

15. Zellzusammensetzung und plazentale extrazelluläre Matrix (ECM) zur Verwendung in einem Verfahren nach einem der Ansprüche 1 bis 14, wobei das Ablagern durch Tintenstrahldruck erreicht wird.

16. Zellzusammensetzung und plazentale extrazelluläre Matrix (ECM) zur Verwendung in einem Verfahren nach Anspruch 1, wobei die Oberfläche nicht Haut, Epidermis oder Dermis ist.

## Revendications

1. Composition cellulaire et matrice extracellulaire (MEC) placentaire destinés à être utilisés dans un procédé de traitement du corps humain ou animal, dans lequel le procédé comprend la formation d'un tissu tridimensionnel comprenant le dépôt de la composition cellulaire et de la MEC placentaire sur une surface dans ou sur un sujet humain ou animal, où la composition cellulaire comprend des cellules productrices d'insuline, et dans lequel ladite composition cellulaire et ladite MEC sont toutes deux imprimées sur ladite surface.

2. Composition cellulaire et matrice extracellulaire (MEC) placentaire destinés à être utilisés dans un procédé selon la revendication 1, dans lequel ladite surface est une surface artificielle, un tissu décellularisé ou un organe décellularisé.

3. Composition cellulaire et matrice extracellulaire (MEC) placentaire destinés à être utilisés dans un procédé selon la revendication 2, dans lequel ladite surface artificielle est un dispositif ou une structure prothétique.

4. Composition cellulaire et matrice extracellulaire (MEC) placentaire destinés à être utilisés dans un procédé selon l'une quelconque des revendications 1 à 4, dans lequel ladite surface est une surface sur l'extérieur dudit sujet, ou dans lequel ladite surface est une surface à l'intérieur dudit sujet.

5. Composition cellulaire et matrice extracellulaire (MEC) placentaire destinés à être utilisés dans un procédé selon l'une quelconque des revendications 1 à 5, comprenant le balayage de ladite surface dans ou sur ledit sujet de sorte à former une cartographie de surface, et l'utilisation de ladite cartographie de surface pour guider ledit dépôt, de préférence, dans lequel ledit balayage comprend l'utilisation d'un laser, d'un faisceau d'électrons, de l'imagerie par résonance magnétique, de micro-ondes, de rayons X, ou de la tomodensitométrie.

6. Composition cellulaire et matrice extracellulaire (MEC) placentaire destinés à être utilisés dans un procédé selon l'une quelconque des revendications 1 à 5, comprenant en outre le dépôt d'un hydrogel, d'un polymère synthétique, de ténascine C ou d'un fragment de celle-ci, et/ou d'une composition de titane-aluminium-vanadium (Ti₆Al₄V).

7. Composition cellulaire et matrice extracellulaire (MEC) placentaire destinés à être utilisés dans un procédé selon l'une quelconque des revendications 1 à 6, dans lequel ladite composition cellulaire comprend en outre
(i) des cellules souches mésenchymateuses dérivées de moelle osseuse (CSM-MO) ;
(ii) des cellules souches placentaires CD34-, CD10+, CD105+, CD200+ adhérentes à un plastique de culture tissulaire ;
(iii) des cellules souches embryonnaires, des cellules souches pluripotentes induites, des cellules souches mésenchymateuses, des cellules souches mésenchymateuses dérivées de moelle osseuse, des cellules stromales mésenchymateuses dérivées de moelle osseuse, des cellules souches placentaires adhérentes à un plastique de culture tissulaire (PDAC), des cellules souches du cordon ombilical, des cellules souches du liquide amniotique, des cellules adhérentes dérivés d'amnios (AMDAC), des cellules adhérentes placentaires ostéogéniques (OPAC), des cellules souches du tissu adipeux, des cellules souches limbiques, des cellules souches de la pulpe dentaire, des myoblastes, des cellules progénitrices endothéliales, des cellules souches neuronales, des cellules souches dérivées de dents exfoliées, des cellules souches des follicules pileux, des cellules souches dermiques, des cellules souches dérivées par parthénogenèse, des cellules souches reprogrammées, des cellules adhérentes dérivées d'amnios, ou des cellules souches SP (« side population ») ; et/ou
(iv) des cellules différenciées.

8. Composition cellulaire et matrice extracellulaire (MEC) placentaire destinés à être utilisés dans un procédé selon la revendication 7, dans lequel lesdites cellules différenciées comprennent des cellules endothéliales, des cellules épithéliales, des cellules dermiques, des cellules endodermiques, des cellules mésodermiques, des fibroblastes, des ostéocytes, des chondrocytes, des cellules tueuses naturelles, des cellules dendritiques, des cellules hépatiques, des cellules pancréatiques, ou des cellules stromales ;
des cellules muqueuses des glandes salivaires, des cellules séreuses des glandes salivaires, des cellules des glandes de von Ebner, des cellules des glandes mammaires, des cellules des glandes lacrymales, des cellules des glandes cérumineuses, des cellules sombres des glandes sudoripares eccrines, des cellules claires des glandes sudoripares eccrines, des cellules des glandes sudoripares apocrines, des cellules des glandes de Moll, des cellules des glandes sébacées, des cellules des glandes de Bowman, des cellules des glandes de Brunner, des cellules des vésicules séminales, des cellules de la glande prostatique, des cellules des glandes bulbo-urétrales, des cellules de la glande de Bartholin, des cellules de la glande de Littré, des cellules de l'endomètre utérin, des cellules caliciformes isolées, des cellules de la muqueuse tapissant l'estomac, des cellules zymogènes des glandes gastriques, des cellules oxyntiques des glandes gastriques, des cellules acinaires pancréatiques, des cellules de Paneth, des pneumocytes de type II, des cellules de Clara,
des somatrotopes, des lactotropes, des thyrotropes, des gonadotropes, des corticotropes, des cellules de l'hypophyse intermédiaire, des cellules neurosécrétoires magnocellulaires, des cellules intestinales, des cellules du tractus respiratoire, des cellules épithéliales de la thyroïde, des cellules parafolliculaires, des cellules des glandes parathyroïdes, des cellules principales de la parathyroïde, des cellules oxyphiles, des cellules des glandes surrénales, des cellules chromaffines, des cellules de Leydig, des cellules de la thèque interne, des cellules du corps jaune, des cellules lutéales de la granulosa, des cellules lutéales de la thèque, des cellules juxtaglomérulaires, des cellules de la macula densa, des cellules péripolaires, des cellules mésangiales,
des cellules endothéliales fenêtrées des vaisseaux sanguins et du système vasculaire lymphatique, des cellules endothéliales continues des vaisseaux sanguins et du système vasculaire lymphatique, des cellules endothéliales spléniques des vaisseaux sanguins et du système vasculaire lymphatique, des cellules synoviales, des cellules séreuses (tapissant les cavités péritonéale, pleurale, et péricardique), des cellules squameuses, des cellules columnaires, des cellules sombres, des cellules de la membrane vestibulaire (tapissant l'espace endolymphatique de l'oreille), des cellules basales de la strie vasculaire, des cellules marginales de la strie vasculaire (tapissant l'espace endolymphatique de l'oreille), des cellules de Claudius, des cellules de Boettcher, des cellules des plexus choroïdes, des cellules squameuses de la pie-mère et l'arachnoïde, des cellules de l'épithélium ciliaire pigmentaire, des cellules de l'épithélium ciliaire non pigmentaire, des cellules endothéliales cornéennes, des cellules intercalaires,
des cellules ciliées du tractus respiratoire, des cellules ciliées de l'oviducte, des cellules ciliées de l'endomètre utérin, des cellules ciliées du rete testis, des cellules ciliées du canal efférent, des cellules ciliées de l'épendyme,
des kératinocytes épidermiques, des cellules basales épidermiques, des kératinocytes des ongles des mains et des ongles des pieds, des cellules basales du lit unguéal, des cellules médullaires de la tige pilaire, des cellules corticales de la tige pilaire, des cellules cuticulaires de la tige pilaire, des cellules cuticulaires de la gaine de la racine pilaire, des cellules de la couche de Huxley de la gaine de la racine pilaire, des cellules de la couche de Henle de la gaine de la racine pilaire, des cellules externes de la gaine de la racine pilaire, des cellules de la matrice pilaire,
des cellules épithéliales de surface de l'épithélium squameux stratifié, des cellules basales de l'épithélium, des cellules de l'épithélium urinaire,
des cellules ciliées auditives internes de l'organe de Corti, des cellules ciliées auditives externes de l'organe de Corti, des cellules basales de l'épithélium olfactif, des neurones sensoriels primaires sensibles au froid, des neurones sensoriels primaires sensibles à la chaleur, des cellules de Merkel de l'épiderme, des neurones récepteurs olfactifs, des neurones sensoriels primaires sensibles à la douleur, des cellules photoréceptrices en bâtonnet, des cellules photoréceptrices de cônes sensibles au bleu, des cellules photoréceptrices de cônes sensibles au vert, des cellules photoréceptrices de cônes sensibles au rouge, des neurones sensoriels primaires proprioceptifs, des neurones sensoriels primaires du toucher, des cellules du corpuscule carotidien de type I, des cellules du corpuscule carotidien de type II (capteur du pH sanguin), des cellules ciliées de type I de l'appareil vestibulaire de l'oreille (accélération et gravité), des cellules ciliées de type II de l'appareil vestibulaire de l'oreille, des cellules des papilles gustatives de type I
des cellules neurales cholinergiques, des cellules neurales adrénergiques, des cellules neurales peptidergiques,
des cellules des piliers internes de l'organe de Corti, des cellules des piliers externes de l'organe de Corti, des cellules des phalanges internes de l'organe de Corti, des cellules des phalanges externes de l'organe de Corti, des cellules de la bordure de l'organe de Corti, des cellules de Hensen de l'organe de Corti, des cellules de soutien de l'appareil vestibulaire, des cellules de soutien des papilles gustatives, des cellules de soutien de l'épithélium olfactif, des cellules de Schwann, des cellules satellites, des cellules gliales entériques,
des astrocytes, des neurones, des oligodendrocytes, des neurones en fuseau,
des cellules épithéliales de la lentille antérieure, des cellules fibreuses de la lentille contenant le cristallin,
des hépatocytes, des adipocytes, des adipocytes blancs, des adipocytes bruns, des lipocytes hépatiques,
des cellules pariétales des glomérules rénaux, des podocytes des glomérules rénaux, des cellules de la bordure en brosse des tubules rénaux proximaux, des cellules du segment grêle de l'anse de Henlé, des cellules des tubules rénaux distaux, des cellules des tubes collecteurs rénaux, des pneumocytes de type I, des cellules canalaires pancréatiques, des cellules des canaux non striés, des cellules canalaires, des cellules de la bordure en brosse intestinale, des cellules des canaux striés des glandes exocrines, des cellules épithéliales de la vésicule biliaire, des cellules non ciliées des canaux efférents, des cellules principales de l'épididyme, des cellules basales de l'épididyme,
des cellules épithéliales des améloblastes, des cellules épithéliales du planum semilunatum, des cellules épithéliales interdentaires de l'organe de Corti, des fibroblastes du tissu conjonctif lâche, des kératocytes cornéens, des fibroblastes du tendon, des fibroblastes du tissu réticulaire de la moelle osseuse, des fibroblastes non-épithéliaux, des péricytes, des cellules du noyau pulpeux, des cémentoblastes/cémentocytes, des odontoblastes, des odontocytes, des chondrocytes du cartilage hyalin, des chondrocytes du fibrocartilage, des chondrocytes du cartilage élastique, des ostéoblastes, des ostéocytes, des ostéoclastes, des cellules ostéoprogénitrices, des hyalocytes, des cellules stellaires (oreille), des cellules stellaires hépatiques (cellules de Ito), des cellules stellaires pancréatiques,
des cellules rouges des muscles squelettiques, des cellules blanches des muscles squelettiques, des cellules intermédiaires des muscles squelettiques, des cellules à sac nucléaire du fuseau musculaire, des cellules à chaîne nucléaire du fuseau musculaire, des cellules satellites, des cellules musculaires cardiaques ordinaires, des cellules musculaires cardiaques nodales, des cellules des fibres de Purkinje, des cellules musculaires lisses, des cellules myoépithéliales de l'iris, des cellules myoépithéliales des glandes exocrines,
des réticulocytes, des mégacaryocytes, des monocytes, des macrophages du tissu conjonctif, des cellules de Langerhans épidermiques, des cellules dendritiques, des cellules microgliales, des neutrophiles, des éosinophiles, des basophiles, des mastocytes, des cellules T auxiliaires, des cellules T suppressives, des cellules T cytotoxiques, des cellules T tueuses naturelles, des cellules B, des cellules tueuses naturelles,
des mélanocytes, des cellules de l'épithélium pigmentaire rétinien, des cellules de Sertoli, des cellules épithéliales thymiques, et/ou des cellules rénales interstitielles.

9. Composition cellulaire et matrice extracellulaire (MEC) placentaire destinés à être utilisés dans un procédé selon l'une quelconque des revendications 1 à 8, dans lequel les cellules dans ladite composition cellulaire sont des cellules de culture primaire, de préférence issues dudit sujet ; ou des cellules qui ont été mises en culture *in vitro.*

10. Composition cellulaire et matrice extracellulaire (MEC) placentaire destinés à être utilisés dans un procédé selon l'une quelconque des revendications 1 à 9, dans lequel lesdites cellules ont été génétiquement manipulées pour produire une protéine ou un polypeptide non naturellement produit(e) par la cellule, ou ont été génétiquement manipulées pour produire une protéine ou un polypeptide en une quantité supérieure à celle naturellement produite par la cellule, où ladite composition cellulaire comprend des cellules différenciées.

11. Composition cellulaire et matrice extracellulaire (MEC) placentaire destinés à être utilisés dans un procédé selon la revendication 10, dans lequel 1 x 10⁶ desdites cellules produit au moins 1,0 à 10 µM de ladite protéine dans une culture *in vitro* dans un milieu de croissance pendant 24 heures.

12. Composition cellulaire et matrice extracellulaire (MEC) placentaire destinés à être utilisés dans un procédé selon la revendication 10, dans lequel ladite protéine est une protéine absente ou déficiente chez un sujet qui souffre d'un trouble ou d'une maladie génétique, de préférence où :
ladite maladie génétique est l'hypercholestérolémie familiale et ladite protéine est le récepteur des lipoprotéines de basse densité (LDLR) ;
ladite maladie génétique est la maladie polykystique des reins, et ladite protéine est la polycystine-1 (PKD1), PKD-2 ou PKD3 ; ou
ladite maladie génétique est la phénylcétonurie et ladite protéine est la phénylalanine hydroxylase.

13. Composition cellulaire et matrice extracellulaire (MEC) placentaire destinés à être utilisés dans un procédé selon la revendication 1, dans lequel ladite surface est préparée en déposant une composition comprenant une biomolécule sur ladite surface avant ladite impression, de préférence dans lequel ladite biomolécule est ou comprend un type de collagène, un type de fibronectine, un type de laminine, ou un adhésif tissulaire ; ou
dans lequel ladite surface est préparée en recouvrant l'intégralité ou une partie de ladite surface avec un tissu décellularisé, de préférence,
dans lequel ledit tissu décellularisé est une membrane amniotique décellularisée, un diaphragme décellularisé, de la peau décellularisée, ou un fascia décellularisé.

14. Composition cellulaire et matrice extracellulaire (MEC) placentaire destinés à être utilisés dans un procédé selon l'une quelconque des revendications 1 à 13, dans lequel ladite surface est une surface du coeur, d'un vaisseau sanguin, de peau, du foie, du pancréas, du poumon, du rein, de la thyroïde, d'une section d'intestin, de l'estomac, de la trachée, de l'oesophage, ou du duodénum chez ledit sujet ; ou dans lequel ladite surface est une peau lésée.

15. Composition cellulaire et matrice extracellulaire (MEC) placentaire destinés à être utilisés dans un procédé selon l'une quelconque des revendications 1 à 14, dans lequel ledit dépôt est réalisé par une impression jet d'encre.

16. Composition cellulaire et matrice extracellulaire (MEC) placentaire destinés à être utilisés dans un procédé selon la revendication 1, dans lequel ladite surface n'est pas de la peau, de l'épiderme, ou du derme.
